# EUROPÄISCHE PATENTSCHRIFT

(11) **EP 4 523 628 B1**
(45) Veröffentlichungstag und Bekanntmachung des Hinweises auf die Patenterteilung: **28.10.2026**
(21) Anmeldenummer: 23197597.0
(22) Anmeldetag: 15.09.2023
(51) Int. Cl.: A61B 6/00

(54) **TECHNIK ZUM BILDVERARBEITENDEN KOMPENSIEREN EINES RÖNTGENGITTERSCHATTENS OHNE VERWENDUNG EINER ZUSÄTZLICHEN MECHANISCHEN AKTORIK**
TECHNIQUE FOR IMAGE PROCESSING COMPENSATION OF AN X-RAY SHADOW WITHOUT THE USE OF AN ADDITIONAL MECHANICAL ACTUATOR
TECHNIQUE DE COMPENSATION DE TRAITEMENT D'IMAGE D'OMBRE À GRILLE DE RAYONS X SANS UTILISER D'UN ACTIONNEUR MÉCANIQUE SUPPLÉMENTAIRE

(43) Veröffentlichungstag der Anmeldung: 19.03.2025
(73) Patentinhaber: Siemens Healthineers AG, 91301 Forchheim (DE)
(72) Erfinder: Friedrich, Stefan, 96135 Stegaurach (DE); Zeidler, Josef, 95615 Marktredwitz (DE)
(74) Vertreter: Siemens Healthineers Patent Attorneys

(56) Entgegenhaltungen:
- DE-A1- 102016 210 529
- KR-A- 20210 004 085
- US-A1- 2020 258 222

## Beschreibung

Die Erfindung betrifft eine Technik zum Kompensieren (insbesondere in Echtzeit) eines Schattens eines Röntgenstreustrahlenabsorptionsgitters in einem Röntgenbild, das mit einem schwenkbar montierten Röntgengerät aufgenommen wird. Insbesondere wird ein Verfahren, eine Rechenvorrichtung, ein System umfassend die Rechenvorrichtung und ein Computerprogrammprodukt bereitgestellt.

Für die Generierung eines Röntgenbildes werden in den Strahlengang zwischen dem Röntgenstrahler und dem Röntgendetektor üblicherweise Röntgenstreustrahlenabsorptionsgitter (auch als Röntgengitter, Streustrahlenabsorptionsgitter und/oder Streustrahlenraster bezeichnet) eingebracht. Somit wird die Streustrahlung mit einem Streustrahlenraster vor dem Bildempfänger herausgefiltert, um eine Erhöhung der Bildqualität zu erreichen. Neuartige, insbesondere additive Verfahren ermöglichen die Fertigung von Streustrahlenrastern mit hohen Aspektverhältnissen (und/oder hoher Höhe der Struktur relativ zur Strukturperiode), sogenannte Super-Raster, "Smartgrids", oder Fokussierte Raster.

Bei den Fokussierten Rastern (auch: "Smartgrids") sind die Lamellen nicht nur höher ausgeführt als für herkömmliche Streustrahlenraster, um eine bessere Filterung zu erreichen, sondern auch in Richtung des Strahlermittelpunkts gerichtet, um einen großen Anteil der Nutzstrahlung zu erhalten.

In konventionellen Angiographiesystemen sind die neuartigen Fokussierten Raster (die auch als Röntgengitter, oder kurz Gitter, bezeichnet werden können) jedoch nicht direkt verwendbar, weil bei Bewegungen durch elastische Verformungen (auch: Deformationen) im Gerät bzw. der Gantry der Brennpunkt des Röntgenstrahlers stark auswandert und dies zu Abschattungen und in der Bildgebung sichtbaren Artefakten führt.

Die Gitter müssen herkömmlicherweise untereinander und zum Röntgendetektor exakt ausgerichtet sein, da die Artefakte, die aufgrund der mechanischen Elastizität und der damit einhergehenden Abschattungen entstehen, bisher nicht herausgerechnet werden können.

Um "Smartgrids" trotz der herkömmlichen Nachteile (insbesondere Abschattungen und/oder Artefakte), beispielsweise in Angiographiesystemen, einsetzen zu können und die Vorteile (insbesondere höhere Trennschärfe bzgl. Streustrahlung und/oder Reduktion der Strahlenbelastung von Patienten und medizinischem Personal) zu gewinnen, wird herkömmlicherweise eine aktive, mechanische Kompensation in Betracht gezogen. Dabei wird beispielsweise der komplette Detektor kippbar ausgeführt, was jedoch einen erhöhten Fertigungsaufwand, ein höheres Gewicht, beispielsweise durch weitere Gelenke und/oder zusätzliche Aktorik, und/oder neue Fehlerquellen und/oder Verschleißstellen nach sich zieht. Alternativ oder ergänzend wird, beispielsweise in dem Dokument DE 10 2016 210 529 A1 [1], auf Seite des Detektors ein aufwändiger mehrachsig betriebener Mechanismus verwendet, um die aufgrund der Elastizität des Arms (insbesondere C-Bogens) entstehenden Verschiebungen durch Feinpositionierung des Detektorgitters kinematisch zu kompensieren.

Die Druckschrift US 2020/0258222A1 beschreibt ein Verfahren zum Training einer Funktion eines Röntgensystems mit Positioniermechanismus, Detektor und Streustrahlenraster. Die Detektorposition wird vielfach verändert, wobei der Mechanismus durch Eigengewicht verzogen oder verdreht wird und in jeder Position mindestens ein Röntgenbild aufgenommen wird. Mittels Machine Learning werden die durch das Raster erzeugten Artefakte aus allen Bildern gelernt und später korrigiert.

Die Druckschrift KR 10-2021-0004085 beschreibt ein KIbasiertes Verfahren zur Entfernung von Grid-Linien und Moiré-Artefakten aus Röntgenbildern, wobei synthetische und reale Trainingsdaten sowie frequenzbasierte Filter und eine U-Net-Architektur verwendet werden. Die Verarbeitung erfolgt blockweise, sodass auch große Bilder effizient und speicherschonend bearbeitet werden können.

Die Druckschrift DE 10 2016 210 529 A1 beschreibt eine mechanische Vorrichtung zur spielfreien drehbaren Lagerung von Röntgengittern, insbesondere für Phasenkontrastbildgebung. Die Konstruktion umfasst Drehrahmen mit Biegeelementen wie Fließgelenken oder Federscharnieren, eine kardanische Aufhängung sowie motorische Linearantriebe für exakte Kipp- und Kantbewegungen. Dadurch wird eine sehr hohe Genauigkeit bei der Ausrichtung des Gitters erreicht.

Es ist daher ein Ziel der vorliegenden Erfindung, eine Lösung für eine verbesserte Bildqualität eines mit einem Röntgenstreustrahlenabsorptionsgitter aufgenommenen Röntgenbilds, insbesondere bei Verwendung eines Fokussierten Rasters, ohne die Notwendigkeit einer zusätzlichen Aktorik und/oder in Leichtbauweise, insbesondere in Echtzeit und/oder zur Laufzeit, bereitzustellen. Alternativ oder ergänzend besteht die Aufgabe, eine Bildqualität bei elastischen Verformungen des Röntgengeräts zwischen Röntgenquelle und Detektor, insbesondere in Echtzeit und/oder zur Laufzeit, zu verbessern.

Diese Aufgabe wird gelöst durch ein Verfahren zum Kompensieren (insbesondere in Echtzeit) eines Schattens eines Röntgenstreustrahlenabsorptionsgitters (kurz: Röntgengitters) in einem Röntgenbild, das mit einem schwenkbar montierten Röntgengerät aufgenommen wird. Die Aufgabe wird ferner gelöst durch eine Rechenvorrichtung, durch ein System, durch ein Computerprogramm (und/oder ein Computerprogrammprodukt) und/oder durch ein computerlesbares Medium gemäß den beigefügten unabhängigen Ansprüchen. Vorteilhafte Aspekte, Merkmale und Ausführungsformen sind in den abhängigen Ansprüchen und in der folgenden Beschreibung zusammen mit Vorteilen beschrieben.

Im Folgenden wird die erfindungsgemäße Lösung in Bezug auf das beanspruchte Verfahren zum Kompensieren (insbesondere in Echtzeit) eines Schattens eines Röntgenstreustrahlenabsorptionsgitters in einem Röntgenbild, das mit einem schwenkbar montierten Röntgengerät aufgenommen wird, sowie in Bezug auf die beanspruchte Rechenvorrichtung beschrieben. Merkmale, Vorteile oder alternative Ausführungsformen hierin können den anderen beanspruchten Gegenständen (z.B. dem System, dem Computerprogramm oder einem Computerprogrammprodukt) zugeordnet werden, und umgekehrt. Mit anderen Worten: Die Ansprüche für die Rechenvorrichtung und/oder das System, umfassend die Rechenvorrichtung können durch Merkmale verbessert werden, die im Zusammenhang mit dem Verfahren beschrieben oder beansprucht werden und umgekehrt. In diesem Fall werden die funktionalen Merkmale des Verfahrens durch Struktureinheiten der Recheneinheit und/oder des Systems verkörpert, und umgekehrt.

Gemäß einem Verfahrensaspekt wird ein (insbesondere computerimplementiertes) Verfahren zum Kompensieren (insbesondere in Echtzeit) eines Schattens eines Röntgenstreustrahlenabsorptionsgitters in einem Röntgenbild bereitgestellt. Das Röntgenbild wird mit einem schwenkbar montierten Röntgengerät aufgenommen.

Das Verfahren umfasst einen Schritt des Empfangens von Bewegungszustandsdaten, die auf einen Bewegungszustand eines Röntgengeräts hinweisen. Das Röntgengerät ist auf einem schwenkbaren Arm montiert ist. Der Bewegungszustand ist einem Zeitpunkt eines Aufnehmens (auch: Bildgebung) eines Röntgenbilds mittels des Röntgengeräts zugeordnet.

Das Verfahren umfasst ferner einen Schritt des Bestimmens einer Position eines Röntgenstreustrahlenabsorptionsgitters, das am Detektor des Röntgengeräts (auch: Röntgendetektor) angeordnet ist. Die Position des Röntgenstreustrahlenabsorptionsgitters umfasst eine Position relativ zur Röntgenquelle (auch: Röntgenstrahler; kurz: Strahler) des Röntgengeräts.

Das Bestimmen wird in Abhängigkeit der empfangenen Bewegungszustandsdaten mittels eines (insbesondere datengetriebenen) Funktionsapproximators ausgeführt.

Das Verfahren umfasst weiterhin einen Schritt des Kompensierens (insbesondere in Echtzeit) eines Schattens des Röntgenstreustrahlenabsorptionsgitters auf dem aufgenommenen Röntgenbild. Das Kompensieren umfasst ein Berücksichtigen der bestimmten Position des Röntgenstreustrahlenabsorptionsgitters in dem aufgenommenen Röntgenbild.

Mittels der erfindungsgemäßen Technik können relative Verschiebungen zwischen den Positionen von Röntgenquelle und Detektor (und/oder dem am Detektor angebrachten Röntgenstreustrahlenabsorptionsgitter, kurz: Röntgengitter) des Röntgengeräts, insbesondere ohne zusätzliche Aktorik zum Fein-Einstellen oder Korrigieren der Detektorposition relativ zur Röntgenquelle, in einer Bildverarbeitung kompensiert werden. Dadurch können Abschattungen des primären Röntgenstrahls durch eine verschobene Position des Röntgengitters in dem aufgenommenen Röntgenbild (auch: Radiographie und/oder Röntgenaufnahme) kompensiert werden. Alternativ oder ergänzend kann mittels der erfindungsgemäßen Technik eine Qualität, beispielsweise eine Schärfe und/oder Detailgenauigkeit, einer Röntgenaufnahme verbessert werden.

Der schwenkbare Arm kann auch als Gantry (und/oder, insbesondere schwenkbares, Gerüst) bezeichnet werden. Alternativ oder ergänzend kann der schwenkbare Arm einen C-Bogen umfassen.

Die relativen Verschiebungen zwischen Röntgenquelle und Detektor in der Detektorebene können auch als Auslenkungen und/oder Auswanderungen bezeichnet werden.

Durch das Kompensieren, insbesondere in Echtzeit (auch: Echtzeit-Kompensation), und/oder die Verbesserung der Qualität, der Röntgenaufnahme kann eine verbesserte Diagnose und/oder eine gezieltere Behandlung eines Patienten ermöglicht werden. Beispielsweise kann bei einer Röntgenaufnahme, insbesondere einer Angiographie, durch die Verwendung eines Röntgengitters eine höhere Trennschärfe des primären Röntgenstrahls von Streustrahlung erreicht werden, wodurch eine Reduktion der Strahlenbelastung von Patienten und medizinischem Personal ermöglicht wird bei verbesserter Bildqualität.

In Echtzeit kann auch als zur Laufzeit, insbesondere der Röntgenaufnahme (und/oder des Röntgenvorgangs), bezeichnet werden. Alternativ oder ergänzend kann durch das Kompensieren in Echtzeit ermöglicht werden, hochwertige Röntgenbilder (und/oder Radiographien) zeitnah bereitzustellen, insbesondere während der Patient sich noch in Aufnahmeposition befindet. Alternativ oder ergänzend wird durch die Echtzeit-Kompensation eine rasche Beurteilung ermöglicht, ob noch weitere Röntgenbilder (und/oder Radiographien) benötigt werden. Weiterhin alternativ oder ergänzend können durch das Kompensieren in Echtzeit künftige (z.B. mehre Tage oder Wochen später) Folgetermine (z.B. für weitere Aufnahmen, zur Besprechung der Diagnose und/oder zur Besprechung eines Behandlungsplans) vermieden werden.

Alternativ oder ergänzend kann vorteilhafterweise aufgrund der erfindungsgemäßen Technik auf eine (z.B. aufwändige und/oder zusätzliche Masse oder Gewicht beitragende) Aktorik zur realen Korrektur, Fein-Einstellung und/oder zum Justieren der Position des Röntgengitters verzichtet werden.

Schwenken kann translatorische und/oder rotatorische Bewegungen umfassen.

Der schwenkbare Arm (und/oder das schwenkbar montierte Röntgengerät) kann translatorische und/oder rotatorische Freiheitsgrade umfassen. Beispielsweise kann der schwenkbare Arm einen C-Bogen, insbesondere mit fünf unabhängigen Freiheitsgraden, umfassen. Alternativ oder ergänzend kann der schwenkbare Arm weniger als fünf unabhängige Freiheitsgrade (z.B. aufgrund einer Befestigung des Arms in einem Raum, insbesondere einem Röntgenraum, Strahlenschutzraum, Behandlungszimmer und/oder Operationssaal) oder mehr als fünf (insbesondere drei translatorische und drei rotatorische) unabhängige Freiheitsgrade haben.

Das Röntgenstreustrahlenabsorptionsgitter (kurz: Röntgengitter) kann auch als Detektorgitter bezeichnet werden. Das Röntgengitter kann Lamellen (z.B. aus Metallblech, insbesondere aus Blei) umfassen, die sich von einer Grundfläche weg erstrecken.

Die Grundfläche des Röntgengitters kann einer Detektorebene (auch: Gitterebene) entsprechen. Alternativ oder ergänzend kann die Position des Röntgengitters entlang der Detektorebene durch zwei-dimensionale (2D) Koordinaten, beispielsweise (x,y), parametrisierbar sein.

Mittels der 2D Koordinaten, beispielsweise (x,y), kann beschrieben werden, wo das Zentrum des Röntgenstrahls am Detektor auftrifft. Idealerweise kann das Zentrum des Röntgenstrahls (z.B. immer) genau mittig am Detektor auftreffen. Aufgrund der Verbiegungseffekte kann das Zentrum des Röntgenstrahls am Detektor auswandern. Die 2D Koordinaten (x,y) können die Auswanderung beschreiben. Alternativ oder ergänzend kann die Position des Röntgengitters am Detektor fixiert sein.

Eine Ruheposition (auch als neutrale Position oder unverschobene Position bezeichnet) des Röntgengitters kann umfassen, dass Röntgenquelle und Detektor des Röntgengeräts senkrecht übereinander (oder untereinander) angeordnet sind, und dass ein Mittelpunkt und/oder Fokalpunkt (auch: Fokuspunkt) eines von der Röntgenquelle emittierten Röntgenstrahls auf einen vorbestimmten Punkt (vorzugsweise dem Mittelpunkt) des Röntgengitters abgebildet wird.

Das Röntgenbild kann auch als Röntgenaufnahme und/oder als Radiographie bezeichnet werden. Alternativ oder ergänzend kann das Röntgenbild eine zeitliche Abfolge von einzelnen (und/oder instantanen) Röntgenbildern umfassen.

Ein Abstand zwischen Röntgenquelle und Röntgengitter, und/oder ein Abstand zwischen Röntgenquelle und Detektor, kann zwischen 90 Zentimetern (cm) und zwei Metern, beispielsweise 1,2m, betragen.

Der Detektor, an dem das Röntgengitter angeordnet ist, kann eine Gewicht von beispielsweise 50kg umfassen. Alternativ oder ergänzend kann der schwenkbare Arm durch die in den Bewegungsdaten erfassten Bewegungen und/oder den Bewegungszustand elastisch verformt werden.

Eine Breite, und/oder ein Durchmesser, des Röntgengitters kann zwischen zehn Zentimetern (cm) und fünfzig Zentimetern, insbesondere zwischen 20cm und 45cm, betragen.

Alternativ oder ergänzend kann eine Fläche des Röntgengitter zwischen 100cm² und 2500cm², insbesondere zwischen 20cm × 20cm und 45cm × 45cm, umfassen.

Das Röntgengitter kann eine rechteckige, insbesondere eine quadratische, Grundfläche umfassen. Alternativ oder ergänzend kann das Röntgengitter eine runde, ovale, oder kreisförmige Grundfläche umfassen.

Eine Höhe des Röntgengitters kann zwischen 5mm und 2cm umfassen. Alternativ oder ergänzend kann das Verhältnis von Höhe zu Grundfläche des Röntgengitters ein zu zehn (1:10) oder einen Wert von eins zu einer Zahl größer als zehn umfassen.

In einem konventionellen Röntgengitter können die Lamellen sich senkrecht zur Grundfläche erstrecken. Alternativ oder ergänzend können in einem, insbesondere fokussierten (auch als "Smartgrid" bezeichneten), Röntgengitter sich die zentralen Lamellen (zumindest annähernd) senkrecht erstrecken mit zum Rand des Röntgengitters hinzunehmenden Winkeln zur Senkrechten der Grundfläche. Alternativ oder ergänzend können die Lamellen auf einen gemeinsamen Fokuspunkt ausgerichtet (und/oder fokussiert) sein. Der gemeinsame Fokuspunkt kann der Position einer punktförmigen Röntgenquelle (insbesondere ohne durch Positionsverstellungen verursachte relative Verschiebungen von Röntgenquelle und Detektor) umfassen.

Die (insbesondere äußeren) Lamellen des Röntgengitters können bis zu einem Winkel von maximal 20° (und/oder π/9) von der Senkrechten zur Grundfläche des Röntgengitters abweichen.

Das Röntgengitter kann mittels additiver Fertigung hergestellt sein. Alternativ oder ergänzend kann das Röntgengitter ein Metall (insbesondere als Fertigungsmaterial), vorzugsweise Blei, umfassen.

Die Lamellen des Röntgengitters können ein (z.B. rechteckiges) Raster und/oder eine (z.B. sechseckige) Wabenstruktur bilden. Das Raster und/oder die Wabenstruktur können mit einem Röntgenstrahl-durchlässigen Material, beispielsweise Aluminium, gefüllt sein. Durch die Füllung des Rasters und/oder der Waben kann eine Stabilität des Röntgengitters verbessert werden.

Die empfangenen Bewegungszustandsdaten können Sensordaten und/oder Steuerdaten des Röntgengeräts umfassen. Alternativ oder ergänzend können die Bewegungszustandsdaten eine Position und/oder Orientierung des schwenkbaren Arms umfassen. Weiterhin alternativ oder ergänzend können die Bewegungszustandsdaten eine Geschwindigkeit, und/oder eine Beschleunigung einer Positionsänderung und/oder eine Orientierungsänderung des schwenkbaren Arms umfassen.

Die anhand der empfangenen Bewegungszustandsdaten bestimmte Position des am Detektor angeordneten Röntgengitters kann eine relative Verschiebung eines vorbestimmten Punkts (vorzugsweise des Mittelpunkts) des Röntgengitters relativ zum Mittelpunkt, und/oder Fokalpunkt, des von der Röntgenquelle emittierten Röntgenstrahls umfassen.

Die relative Verschiebung zwischen Röntgenstrahler und Detektor kann durch statische Kräfte, und/oder dynamische Kräfte (die insbesondere gleich null sein können, wenn eine Geschwindigkeit gleich und/oder Beschleunigung null ist), verursacht sein. Beispielsweise kann der schwenkbare Arm des Röntgengeräts eine elastisch verformbare Verbindung (insbesondere einen C-Bogen) zwischen der Röntgenquelle und dem Detektor umfassen. Alternativ oder ergänzend kann der schwenkbare Arm (und/oder das Röntgengerät) eine (insbesondere vom Bewegungszustand abhängige) elastisch verformbare Halterung des Detektors umfassend das Röntgengitter umfassen.

Bei einer Abweichung von der Ruheposition kann sich der schwenkbare Arm (und/oder die Verbindung) elastisch verformen, beispielsweise aufgrund der Schwerkraft (auch: Gravitationskraft; kurz: Gravitation). Alternativ oder ergänzend können Trägheit, Zentrifugalkraft und/oder Corioliskraft eine Verformung des schwenkbaren Arms (und/oder der elastisch verformbaren Verbindung) von Röntgenquelle und Detektor bewirken.

Die Gravitation kann eine statisch wirkende, positionsabhängige Kraft auf den schwenkbaren Arm und/oder das Röntgengerät, insbesondere dessen Detektor und Röntgenquelle, umfassen. Alternativ oder ergänzend können dynamische Kräfte (z.B. Trägheitskraft, Zentrifugalkraft, und/oder Corioliskraft) bewegungsabhängig (insbesondere abhängig von einer Geschwindigkeit und/oder Beschleunigung einer Positionsveränderung) auf den schwenkbaren Arm und/oder das Röntgengerät, insbesondere dessen Detektor und Röntgenquelle, wirken.

Der (insbesondere datengetriebene) Funktionsapproximator kann eine Abbildung und/oder Funktion umfassen, die die empfangenen Bewegungszustandsdaten (insbesondere Positionsdaten und/oder Geschwindigkeitsdaten pro unabhängigem Freiheitsgrad des schwenkbaren Arms) mittels einer (insbesondere gespeicherten und/oder vorbestimmten) Funktion auf eine Position (vorzugsweise eine Positionsverschiebung relativ zur Ruheposition) entlang der 2D Detektorebene abbildet.

Das, oder ein jedes, Röntgenbild (und/oder jede beliebige Radiographie) kann als digitale Bilddaten aufgenommen werden.

Das Kompensieren (insbesondere in Echtzeit) der Position des Röntgengitters kann eine Bildbearbeitung, und/oder Korrektur, eines (insbesondere digitalen) Röntgenbilds umfassen. Alternativ oder ergänzend können durch das Röntgengitter verursachte Bildartefakte kompensiert werden und/oder die Qualität des (insbesondere in Echtzeit) aufgenommen Röntgenbilds verbessert werden.

Das Verfahren kann Cloud-basiert ausgeführt werden.

Alternativ oder ergänzend kann das Verfahren auf einem lokalen Prozessor, einer zentralen Verarbeitungseinheit (fachsprachlich: Central Processing Unit, CPU), einem Neuromorphen Prozessor (fachsprachlich: Neuromorphic Processor Unit, und/oder Neural Processing Unit; kurz: NPU) und/oder einem Grafikprozessor (fachsprachlich: Graphics Processing Unit, GPU) ausgeführt werden.

Der schwenkbare Arm kann einen C-Bogen umfassen.

Das Röntgengerät (z.B. mit der elastisch verformbaren Verbindung ausgebildet als schwenkbarer Arm, insbesondere als C-Bogen) kann einen M-dimensionalen Raum der Freiheitsgrade umfassen, z.B. mit M=5, insbesondere beim C-Bogen. Alternativ kann der schwenkbare Arm, insbesondere der C-Bogen, M<5 oder M>5 unabhängige Freiheitsgrade haben.

Alternativ oder ergänzend kann der Bewegungszustand, beispielsweise des C-Bogens, Gelenkwinkel von Achsen, Geschwindigkeiten von Achsen, und/oder Beschleunigungen von Achsen umfassen.

Der Funktionsapproximator kann eine funktionale Abhängigkeit der Koordinaten in der Detektorebene vom Bewegungszustand des schwenkbaren Arms (und/oder des Röntgengeräts) umfassen und/oder parametrisieren. Alternativ oder ergänzend kann der Funktionsapproximator einen digitalen Zwilling des schwenkbaren Arms (und/oder des Röntgengeräts) umfassen.

Mittels des Funktionsapproximators kann der Bewegungszustand (beispielsweise umfassend 2M verallgemeinerte Koordinaten entsprechend verallgemeinerten Positionen und verallgemeinerten Geschwindigkeiten) auf einen 2D Raum abgebildet werden. Der 2D Raum kann der Detektorebene entsprechen.

Gemäß einem Ausführungsbeispiel können diskrete Werte der Freiheitsgrade, und/oder des Bewegungszustands, vorbestimmt sein. Alternativ oder ergänzend kann der Funktionsapproximator zur Abbildung diskreter Werte des Bewegungszustands auf die Position des Röntgengitters ausgebildet sein.

Die Position des am Detektor angeordneten Röntgengitters kann eine Position in einer Detektorebene umfassen. Alternativ oder ergänzend kann die Position des am Detektor angeordneten Röntgengitters eine Position in einer Ebene senkrecht zu einer Fokusrichtung der Röntgenquelle des Röntgengeräts umfassen.

Der Bewegungszustand kann eine Position, insbesondere eine Orientierung im Raum, umfassen. Alternativ oder ergänzend kann der Bewegungszustand eine Geschwindigkeit einer Positionsverstellung (insbesondere pro Freiheitsgrad des schwenkbaren Arms) und/oder eine Beschleunigung der Positionsverstellung (insbesondere pro Freiheitsgrad des schwenkbaren Arms) des Röntgengeräts umfassen.

Gemäß einem Ausführungsbeispiel kann der Bewegungszustand zwei-dimensional (2D) sein und/oder als Koordinaten Position und Geschwindigkeit (z.B. als Positionsänderung pro Zeiteinheit) des Röntgengeräts (z.B. des C-Bogens) umfassen, insbesondere pro (z.B. unabhängigem) Freiheitsgrad des schwenkbaren Arms.

Gemäß einem weiteren Ausführungsbeispiel kann der Bewegungszustand zu äquidistanten Zeitpunkten abgetastete diskrete Positions-Signale (und/oder Positionen) und/oder eine Reihe von ein-dimensionalen (1D) Koordinaten, die der Position des schwenkbaren Arms des Röntgengeräts (z.B. des C-Bogens) zu einem vorbestimmten Zeitpunkt in einer Zeitreihe entsprechen, umfassen.

Die Menge aller Positionen, und optional Geschwindigkeiten (und/oder Beschleunigungen), kann auch als Zustandsraum des Röntgengeräts (und/oder des schwenkbaren Arms) bezeichnet werden.

Der Funktionsapproximator kann eine Abbildung von einem mindestens M-dimensionalen, insbesondere 2M-dimensionalen, Bewegungszustand des Röntgengeräts auf eine 2D Position des am Detektor angeordneten Röntgengitters umfassen. M kann eine Anzahl unabhängiger, insbesondere schwenkbarer, Freiheitsgrade des schwenkbaren Arms des Röntgengeräts umfassen.

Der Funktionsapproximator kann durch tiefes Lernen (fachsprachlich: Deep Learning) bestimmt werden. Alternativ oder ergänzend kann der Funktionsapproximator die Abbildung als Näherung (auch: Approximation) einer (insbesondere stetigen) Funktion des Bewegungszustands (z.B. für eine Menge vorbestimmter diskreter Bewegungszustände) umfassen.

In einem Ausführungsbeispiel kann der Funktionsapproximater eine Mehrzahl von vorbestimmten Stützstellen in einem (insbesondere M-dimensionalen oder 2M-dimensionalen) Raum der Bewegungszustände umfassen. Beispielsweise kann die Abbildung auf die 2D Position des Röntgengitters von einer oder mehrerer Stützstellen (z.B. linear) interpoliert werden.

Alternativ oder ergänzend kann der Funktionsapproximator durch künstliche Intelligenz (KI), insbesondere ein neuronales Netzwerk (NN), bestimmt sein.

Der Funktionsapproximator kann ein trainiertes NN umfassen.

Eine Anzahl Neuronen einer Eingangsschicht des NN kann ein ganzzahliges Vielfaches einer Anzahl M unabhängig positionsverstellbarer Freiheitsgrade des Röntgengeräts umfassen. Das ganzzahlige Vielfache kann vorzugsweise gleich eins oder zwei sein.

Eine Ausgangsschicht des NN kann zwei Neuronen umfassen, deren Ausgabe jeweils einer (insbesondere unabhängigen, z.B. zueinander senkrechten) Richtung einer Detektorebene zugeordnet ist.

Ein Neuron einer Schicht eines NN kann auch als Knoten der Schicht des NN bezeichnet werden.

Die Neuronen der Eingangsschicht können dazu ausgebildet sein, eine verallgemeinerte Ortskoordinate (z.B. einen Winkel eines rotatorischen Freiheitsgrads) pro Freiheitsgrad, und optional eine verallgemeinerte Geschwindigkeit (z.B. eine Winkelgeschwindigkeit des rotatorischen Freiheitsgrads) pro Freiheitsgrad, zu empfangen. Alternativ oder ergänzend können die Neuronen der Eingangsschicht dazu ausgebildet sein, eine Zeitreihe der verallgemeinerten Ortskoordinate zu empfangen.

Mittels der Zeitreihe kann eine Zeitreihenanalyse und/oder Zeitreihenprädiktion (fachsprachlich: time series prediction) ausgeführt werden, insbesondere ohne eine unabhängige Messung einer (z.B. verallgemeinerten) Geschwindigkeit oder (z.B. verallgemeinerten) Beschleunigung.

Die Neuronen der Ausgangsschicht können dazu ausgebildet sein, Koordinaten einer Verschiebung des Röntgengitters in der Detektorebene auszugeben.

Das NN kann mehrere Schichten (fachsprachlich: Layers) umfassen. Beispielsweise kann das NN eine Eingangsschicht (fachsprachlich: Input Layer), eine oder mehrere verborgene Schichten (fachsprachlich: Hidden Layers) und eine Ausgabeschicht (fachsprachlich: Output Layer) umfassen.

Verbindungen zwischen (z.B. Neuronen von) aufeinanderfolgenden Schichten (beispielsweise zwischen benachbarten verborgenen Schichten) können Übertragungsfunktionen (fachsprachlich: Transfer Function) umfassen. Eine oder jede Übertragungsfunktion kann beispielsweise eine Logistische Funktion (fachsprachlich: logistic function), eine lineare Funktion, und/oder eine Sigmoid-Funktion umfassen.

Das NN kann ein tiefes NN (fachsprachlich: Deep NN; kurz: DNN) umfassen. Alternativ oder ergänzend kann das NN ein faltendes NN (fachsprachlich: convolutional NN, kurz: CNN), insbesondere ein tiefes CNN, umfassen. Weiterhin alternativ oder ergänzend kann das NN ein tiefes CNN umfassen.

Das NN kann mittels überwachtem Training trainiert sein. Ein Trainingsdatensatz kann Eingangsdaten umfassen, die Bewegungszustandsdaten des Röntgengeräts umfassen. Der Trainingsdatensatz kann alternativ oder ergänzend Ausgangsdaten umfassen, die eine vom Bewegungszustand des Röntgengeräts abhängige Position des Röntgengitters umfassen.

Die vom Bewegungszustand des Röntgengeräts abhängige Position des Röntgengitters kann auch als Verschiebung in der Detektorebene (oder Verschiebung der Detektorebene) bezeichnet werden.

Die Position in den Ausgangsdaten eines Trainingsdatensatzes kann (z.B. mittels Laser) gemessen sein.

Die Trainingsdatensätze können reale Trainingsdatensätze umfassen. Die realen Trainingsdatensätze können Messwerte hinweisend auf den Bewegungszustand und/oder auf die Position des Röntgengitters umfassen.

Alternativ oder ergänzend können die Trainingsdatensätze synthetische Trainingsdatensätze umfassen. Die synthetischen Trainingsdatensätze können synthetisch generierte Daten hinweisend auf den Bewegungszustand und/oder eine mittels eines Simulationsmodells bestimmte Position des Röntgengitters umfassen.

Das Simulationsmodell kann ein elastisches Mehrkörperdynamikmodell des schwenkbaren Arms des Röntgengeräts, insbesondere des C-Bogens, umfassen.

Die synthetischen Trainingsdatensätze können auch als simulierte Trainingsdatensätze (kurz auch: Trainingsdaten) bezeichnet werden. Vorteilhaft an der Verwendung von synthetischen Trainingsdatensätzen kann eine Verfügbarkeit einer großen Menge von Trainingsdaten sein. Nachteilhaft kann sein, dass eine Güte der synthetischen Trainingsdatensätze durch die Güte des Simulationsmodells limitiert ist.

Die reale Trainingsdatensätze können Labormessungen, z.B. mittels eines Lasertrackers, umfassen, um Verschiebungen auf der Detektorebene zu messen. Vorteilhaft an realen Trainingsdatensätzen ist, dass nicht (oder nicht ausreichend) simulierte Effekte, z.B. fertigungsbedingte Toleranzen des schwenkbaren Arms (und/oder des Röntgengeräts), miteinbezogen werden. Nachteilig an realen Trainingsdatensätzen kann sein, dass es aufwändig ist, einen großen Satz an realen Trainingsdaten zusammenzustellen.

Es ist möglich, sowohl reale als auch synthetische Trainingsdatensätze für das Training des NN zu verwenden.

Alternativ oder ergänzend kann das NN zunächst für eine vorbestimmten Gerätetypen (insbesondere von schwenkbaren Armen und/oder Röntgengeräten) trainiert (beispielsweise mittels synthetischer Trainingsdatensätze) und anschließend für ein ausgewähltes (und/oder individuelles) Röntgengerät nachtrainiert (und/oder ge-fine-tuned) werden anhand von realen Trainingsdaten des ausgewählten (und/oder individuellen) Röntgengeräts.

Durch das Nachtraining (und/oder Fine Tuning) kann die Bildqualität für jedes hergestellte Röntgengerät (beispielsweise vor Auslieferung) individuell verbessert werden.

Das NN kann eine Feedforward NN (FNN) Architektur umfassen. Optional kann das FNN Schichten als Multi-Layer-Perzeptron (MLP) umfassen.

In einem ersten Ausführungsbeispiel umfassen die Eingangsdaten des FNN (insbesondere umfassend MLP-Schichten), insbesondere sowohl in einer Trainingsphase als auch in einer Inferenzphase, als Bewegungszustand eine Position und eine Geschwindigkeit der Positionsverstellung pro unabhängigem Freiheitsgrad des schwenkbaren Arms (und/oder des Röntgengeräts).

In einem zweiten Ausführungsbeispiel umfassen die Eingangsdaten des FNN (insbesondere umfassend MLP-Schichten), insbesondere sowohl in einer Trainingsphase als auch in einer Inferenzphase, eine Zeitreihe (fachsprachlich: Time Series) von Positionen (insbesondere ohne Geschwindigkeiten als Eingangsdaten). Alternativ oder ergänzend umfasst das zweite Ausführungsbeispiel eine Zeiteinbettung (fachsprachlich: Time Embedding).

Eine Anzahl von Schritten in der Zeitreihe kann als Einbettungstiefe bezeichnet werden und/oder eine Einbettungstiefe umfassen.

Das Training des NN (beispielsweise des FNN) kann eine Fehlerrückführung (fachsprachlich: Backpropagation) umfassen. Alternativ oder ergänzend können Ausgangsdaten auf ein festes (z.B. zeitliches) Intervall mit anschließender Rückskalierung normiert werden.

Alternativ oder ergänzend kann das NN eine Rekurrente NN (RNN) Architektur umfassen. Optional kann das RNN eine Long Short-Term Memory (LSTM) Architektur umfassen.

Das RNN kann eine oder mehrere (z.B. "reine") feedforward Schichten umfassen. Alternativ oder ergänzend kann das NN als RNN bezeichnet werden, sofern mindestens ein rückführendes und/oder speicherndes Neuron in einer (insbesondere verborgenen) Schicht enthalten ist.

Die Eingangsdaten des RNN (insbesondere umfassend die LTSM Architektur) können, insbesondere sowohl in einer Trainingsphase als auch in einer Inferenzphase, als Bewegungszustand eine Position des schwenkbaren Arms (und/oder des Röntgengeräts) umfassen.

Die Architektur des RNN (insbesondere umfassend die LTSM Architektur) kann eine oder mehrere Rückführungen von Neuronen untereinander (und/oder innerhalb des RNN) umfassen.

Das RNN (insbesondere umfassend die LTSM Architektur) kann insbesondere für den Anwendungsfall geeignet sein, falls dynamische Effekte eines Durchbiegens und/oder einer Abschattung der Detektorebene durch das Röntgengitter dominieren. Alternativ oder ergänzend kann auf eine Zeiteinbettung bei Verwendung des RNN (insbesondere umfassend die LTSM Architektur) verzichtet werden.

Eine Trainingsphase des RNN kann länger sein als eine Trainingsphase des FNN.

Das Verfahren kann ferner einen Schritt des Ausgebens des aufgenommenen Röntgenbilds umfassend den (insbesondere in Echtzeit) kompensierten Schatten des Röntgengitters umfassen.

Das Ausgeben des aufgenommenen Röntgenbilds (und/oder der Radiographie) kann eine KI-basierte (z.B. mittels des NN) Bildrekonstruktion umfassen.

Im Schritt des Bestimmens der Position des Röntgengitters kann mittels des (insbesondere datengetriebenen) Funktionsapproximators ein Kalibrierpunkt, und optional ein Kalibrierbild, mittels eines Optimierungsverfahrens bestimmt werden. Das Optimierungsverfahren kann von dem (insbesondere datengetriebenen) Funktionsapproximator ausgeführt werden.

Im Schritt des Kompensierens (insbesondere in Echtzeit) des Schattens des Röntgengitters kann das Kalibrierbild mit dem aufgenommenen Röntgenbild verrechnet werden. Insbesondere kann das Kalibrierbild von dem aufgenommenen Röntgenbild subtrahiert werden.

Das Bestimmen des Kalibrierbilds kann ein Aussuchen und/oder ein Berechnen umfassen. Alternativ oder ergänzend kann das Bestimmen des Kalibrierpunkt eine Auswahl eines (insbesondere besten) Kalibrierpunkts umfassen.

Alternativ oder ergänzend können interpolierte Kalibrierdaten mittels Gewichtung gemäß einer Nähe eines verschobenen Punkts zu einem (insbesondere "ursprünglichen") Punkt (z.B. in der Ruheposition des Röntgengeräts) bestimmt werden. Die Nähe (z.B. für die Gewichtung) des verschobenen Punkts kann proportional sein zu einer Funktion, insbesondere einer positiven Potenz, des inversen Abstands, zum (insbesondere "ursprünglichen") Punkt. Es kann nur ein Punkt mit einem Abstand unterhalb, oder gleich, einem vorbestimmten Schwellenwert (auch: Grenzwert) für die Interpolation verwendet werden.

Durch die Bestimmung des Kalibrierpunkts kann auf weitere Kalibriermessungen verzichtet werden.

Herkömmliche Kalibrierungsmessungen können eine Vielzahl (z.B. Gitter-) Punkte (auch: Kalibrierpunkte) in der Detektorebene, z.B., 5×5=25, 6×6=36 oder 7×7=49, nötig machen. Hierdurch sind herkömmliche Kalibrierungsverfahren üblicherweise nicht Echtzeit-fähig.

Hinsichtlich der Kalibration können mehrere Betriebspunkte (und/oder Kalibrierpunkte) des Röntgenstrahls benutzt werden, so dass zur Laufzeit für (insbesondere alle) relevante(n) Betriebszustände passende Kalibrierbilder vorhanden sind. Insbesondere der Abstand zwischen Strahler und Detektor kann wesentlich sein. Alternativ oder ergänzend können oder müssen, wenn zur Bildgebung unterschiedliche Fokalpunkte verwendet werden, diese unterschiedlichen Fokalpunkte einzeln eingestellt und entsprechend Kalibrierbilder aufgenommen werden. Selbst wenn also der Röntgenstrahl nicht auswandert, sondern immer perfekt mittig am Röntgengitter des Detektors verbleibt, können mehrere Kalibrierbilder benutzt werden.

Eine (z.B. notwendige) Anzahl von Kalibrierpunkten aufgrund der Fokuseinstellung kann einer herkömmlichen Anzahl entsprechen. Alternativ oder ergänzend können Unschärfen aufgrund (z.B. einer geringen Anzahl von) Kalibrierpunkten im Röntgenbild vorhanden sein, insbesondere auch nach dem erfindungsgemäßen Kompensieren des Schattens des Röntgengitters.

Falls eine "Verbiegung" auch in die dritte Richtung am Detektor (insbesondere entlang der Röntgenstrahlrichtung, und/oder entlang einer z-Richtung senkrecht zu Detektorebene in (x,y)-Richtungen) auftritt, können weitere Unterschärfen und/oder Schatten auftreten. Dieser Fall ist jedoch im Allgemeinen praktisch vernachlässigbar.

Abschattungen können vermieden und/oder kompensiert werden, die aufgrund des "Auswanderns" des Strahls vom Zentrum weg (und/oder in x und/oder y-Richtungen) entstehen. Wie und warum (und/oder aufgrund welcher physikalischer Effekte) das Auswandern zustande kommt, ist (beispielsweise für die erfindungsgemäße Technik zum Kompensieren des Schattens des Röntgengitters) unerheblich. Alternativ oder ergänzend wird aufgrund des, insbesondere datengetriebenen, Verfahrens (insbesondere aufgrund des Funktionsapproximators) ein "irgendwie" in Abhängigkeit der physikalischen Zustände (und/oder Bewegungszustandstagen) vorhergesagt.

Um das Wissen über Abschattungen zu nutzen, kann es nötig sein, "bessere" Kalibrierdaten zu verwenden, als wenn man nur einmal (insbesondere in der Mitte des Röntgenbilds und/oder der Detektorebene) kalibrieren würde. Beispielsweise kann ein Raster mit einer Mehrzahl, z.B. 5x5, Kalibrierbildern verwendet werden.

Die Kalibrierdaten können (z.B. im Prinzip) unabhängig von einem echten und/oder gefertigten Arm (insbesondere C-Bogen) erhalten werden, indem eine Vorrichtung verwendet wird, die Strahler und Detektor im genau passenden Abstand zueinander positioniert. Danach kann der Strahler (z.B. minimal) verkippt werden, um den Röntgenstrahl auf einer Position, z.B. bezeichnet mit (1,1), zu erhalten und ein Kalibrierbild aufzunehmen. Danach kann der Strahler (z.B. minimal) verkippt werden auf eine weitere Position, z.B. (1,2). Ein weiteres Kalibrierbild kann aufgenommen werden. Der Vorgang des Verkippens des Strahlers und das Aufnehmen eines Kalibrierbilds können wiederholt werden, bis alle (z.B. vorbestimmten) Kalibrierpunkte berücksichtigt wurden.

Zur Laufzeit kann die tatsächliche und/oder vermutete Position des Röntgenstrahls in der Gitterebene, beispielsweise mittels eines NNs, berechnet und dann aus den vorhandenen Kalibrierdaten eine (insbesondere gut passende) Kompensation der Abschattung errechnet werden.

Mittels der erfindungsgemäßen Technik (beispielsweise umfassend das, insbesondere computer-implementierte, Verfahren) kann eine (z.B. Zusatz-) Aktorik zur physikalischen Nachführung des Röntgengitters vermieden werden. Die (z.B. Zusatz-) Aktorik kann (z.B. herkömmlicherweise) dazu verwendet werden, um die Abschattungseffekte, insbesondere vor dem Aufnehmen des Röntgenbilds und/oder physikalisch, zu kompensieren (z.B. mit nur "einem" Kalibrierpunkt, und/oder durch eine physikalische Verschiebung des Detektors).

Mittels der erfindungsgemäßen Technik kann das Kompensieren des Schattens des Röntgengitters auf eine (insbesondere softwarebasierte) Artefaktreduktion zurückgeführt werden. Die (insbesondere softwarebasierte) Artefaktreduktio kann durch eine hinreichend große Menge an Kalibrierdaten sowie eines echtzeitfähigen "Auswahlverfahrens" ermöglicht werden.

Alternativ oder ergänzend kann mittels des (insbesondere computer-implementierten) Verfahrens auf eine zusätzliche Aktorik zum separaten - und/oder von sonstigen Aktoren des Arms (insbesondere C-Bogens) unabhängigen - Schwenken, und/oder Verschieben, des Detektors (und/oder des Röntgengitters) verzichtet werden.

Gemäß einem Vorrichtungsaspekt wird eine Rechenvorrichtung zum Kompensieren (insbesondere in Echtzeit) eines Schattens eines Röntgenstreustrahlenabsorptionsgitters in einem Röntgenbild, das mit einem schwenkbar montierten Röntgengerät aufgenommen wird, bereitgestellt. Die Rechenvorrichtung umfasst eine Empfangsschnittstelle, die zum Empfangen von Bewegungszustandsdaten, die auf einen Bewegungszustand eines Röntgengeräts hinweisen, ausgebildet ist. Das Röntgengerät ist auf einem schwenkbaren Arm montiert. Der Bewegungszustand ist einem Zeitpunkt eines Aufnehmens eines Röntgenbilds mittels des Röntgengeräts zugeordnet.

Die Rechenvorrichtung umfasst ferner einen (insbesondere datengetriebenen) Funktionsapproximator, der zum Bestimmen einer Position eines Röntgenstreustrahlenabsorptionsgitters, das am Detektor des Röntgengeräts angeordnet ist, ausgebildet ist. Die Position des Röntgenstreustrahlenabsorptionsgitters umfasst eine Position relativ zur Röntgenquelle des Röntgengeräts. Das Bestimmen wird in Abhängigkeit der empfangenen Bewegungszustandsdaten ausgeführt.

Des Weiteren umfasst die Rechenvorrichtung eine Kompensiereinheit, die zum Kompensieren (insbesondere in Echtzeit) eines Schattens des Röntgenstreustrahlenabsorptionsgitters auf dem aufgenommenen Röntgenbild, ausgebildet ist. Das Kompensieren umfasst ein Berücksichtigen der bestimmten Position des Röntgenstreustrahlenabsorptionsgitters in dem aufgenommenen Röntgenbild.

Optional umfasst die Rechenvorrichtung ferner eine Ausgabeschnittstelle, die dazu ausgebildet ist, das aufgenommene Röntgenbild umfassend den (insbesondere in Echtzeit) kompensierten Schatten des Röntgenstreustrahlenabsorptionsgitters auszugeben.

Die Rechenvorrichtung kann dazu ausgebildet sein, einen oder mehrere Schritte des Verfahrens gemäß dem Verfahrensaspekt auszuführen. Alternativ oder ergänzend kann die Rechenvorrichtung eines oder mehrere Merkmale, die im Zusammenhang mit dem Verfahren gemäß dem Verfahrensaspekt offenbart sind, umfassen.

Gemäß einem Systemaspekt wird ein System zum Kompensieren (insbesondere in Echtzeit) eines Schattens eines Röntgenstreustrahlenabsorptionsgitters in einem Röntgenbild, das mit einem schwenkbar montierten Röntgengerät aufgenommen wird, bereitgestellt. Das System umfasst ein oder mehrere jeweils auf einem schenkbaren Arm montierte Röntgengeräte mit je einem Röntgenstreustrahlenabsorptionsgitter. Das System umfasst ferner eine Rechenvorrichtung gemäß den Vorrichtungsaspekt.

Das System kann dazu ausgebildet sein, das Verfahren auszuführen. Die Rechenvorrichtung kann das Verfahren gemäß dem Verfahrensaspekt für jedes der Röntgengeräte (insbesondere getrennt und/oder unabhängig) ausführen.

Gemäß einem weiteren Aspekt wird ein Computerprogrammprodukt bereitgestellt. Das Computerprogrammprodukt umfasst Programmelemente, die eine Rechenvorrichtung (beispielsweise die Rechenvorrichtung gemäß dem Vorrichtungsaspekt) veranlassen, die Schritte des Verfahrens zum Kompensieren (insbesondere in Echtzeit) eines Schattens eines Röntgenstreustrahlenabsorptionsgitters in einem Röntgenbild, das mit einem schwenkbar montierten Röntgengerät aufgenommen wird, gemäß dem Verfahrensaspekt auszuführen, wenn die Programmelemente in einen Speicher der Rechenvorrichtung geladen werden.

Gemäß einem noch weiteren Aspekt wird ein computerlesbares Medium bereitgestellt, auf dem Programmelemente gespeichert sind, die von einer Rechenvorrichtung (beispielsweise der Rechenvorrichtung gemäß dem Vorrichtungsaspekt) gelesen und ausgeführt werden können, um Schritte des Verfahrens zum Kompensieren (insbesondere in Echtzeit) eines Schattens eines Röntgenstreustrahlenabsorptionsgitters in einem Röntgenbild, das mit einem schwenkbar montierten Röntgengerät aufgenommen wird, gemäß dem Verfahrensaspekt durchzuführen, wenn die Programmelemente von der Rechenvorrichtung ausgeführt werden.

Die oben beschriebenen Eigenschaften, Merkmale und Vorteile der vorliegenden Erfindung sowie die Art und Weise, wie sie erreicht werden, werden im Lichte der folgenden Beschreibung und der Ausführungsbeispiele, die im Zusammenhang mit den Zeichnungen näher erläutert werden, klarer und verständlicher. Diese folgende Beschreibung beschränkt die Erfindung nicht auf die enthaltenen Ausführungsformen. Gleiche Komponenten oder Teile können in verschiedenen Figuren mit den gleichen Bezugszeichen versehen sein. Im Allgemeinen sind die Abbildungen nicht maßstabsgetreu.

Es versteht sich, dass eine bevorzugte Ausführungsform der vorliegenden Erfindung auch eine beliebige Kombination der abhängigen Ansprüche oder der obigen Ausführungsformen mit dem jeweiligen unabhängigen Anspruch sein kann.

Diese und andere Aspekte der Erfindung werden aus den nachfolgend beschriebenen Ausführungsformen ersichtlich und werden durch Bezugnahme auf diese erläutert.

### KURZE BESCHREIBUNG DER ZEICHNUNGEN

Fig. 1 ist ein Flussdiagramm eines Verfahrens zum Kompensieren, insbesondere in Echtzeit, eines Schattens eines Röntgengitters in einem Röntgenbild, das mit einem schwenkbar montierten Röntgengerät aufgenommen wird, gemäß einer bevorzugten Ausführungsform der vorliegenden Erfindung;
Fig. 2 ist eine Übersicht über die Struktur und den Aufbau einer Rechenvorrichtung zum Kompensieren, insbesondere in Echtzeit, eines Schattens eines Röntgengitters in einem Röntgenbild, das mit einem schwenkbar montierten Röntgengerät aufgenommen wird, gemäß einer bevorzugten Ausführungsform der vorliegenden Erfindung;
Fig. 3A und 3B zeigen ein konventionelles Röntgengitter mit zu einer Grundfläche senkrechten Lamellen bzw. ein sogenanntes "Smartgrid" mit in Richtung einer Röntgenquelle hin ausgerichteten Lamellen, wobei das erfindungsgemäße Verfahren zum Kompensieren des Schattens insbesondere des "Smartgrids" geeignet ist;
Fig. 4 und 5 zeigen eine aus dem Stand der Technik bekannte Technik zur Vermeidung eines Schattens eines Röntgengitters mittels zusätzlicher Aktorik;
Fig. 6A, 6B und 6C zeigen ein auf einem C-Bogen montiertes Röntgengerät mit je nach Orientierung des C-Bogens unterschiedlichen auf den Röntgendetektor und die Röntgenquelle wirkenden Kräften sowie daraus resultierenden, einen Schatten des Röntgengitter verursachenden, Verschiebungen des Fokuspunkts des Röntgenstrahls auf dem Röntgendetektor;
Fig. 7 zeigt eine perspektivische Ansicht des Röntgendetektors mit Koordinatensystem in der Detektorebene;
Fig. 8 zeigt schematisch einen Weg eines Fokuspunkts eines Röntgenstrahls auf einer Detektorebene, wobei der Weg durch sich ändernde wirkende Kräfte auf den Detektor und die Röntgenquelle eines Röntgengeräts bedingt sein kann; und
Fig. 9A, 9B und 9C zeigen ein Beispiel einer Subtraktion eines Kalibrierbilds von einem aufgenommenen Röntgenbild, um das Röntgengitter aus dem ausgegebenen Röntgenbild herauszurechnen.

Etwaige Bezugszeichen in den Ansprüchen sind nicht als Einschränkung des Anwendungsbereichs zu verstehen.

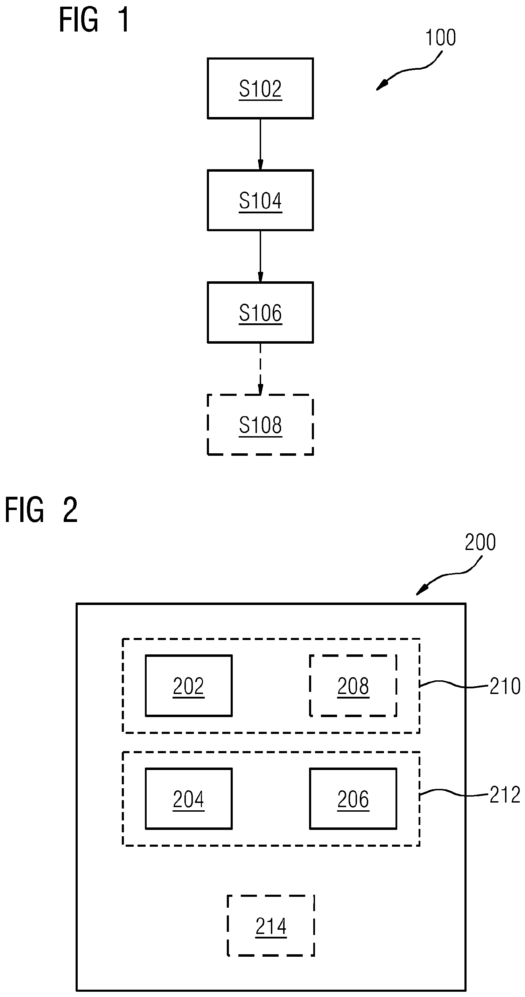

Fig. 1 zeigt schematisch ein beispielhaftes Flussdiagramm eines (insbesondere computer-implementierten) Verfahrens zum Kompensieren (insbesondere in Echtzeit) eines Schattens eines Röntgenstreustrahlenabsorptionsgitters (kurz: Röntgengitter) in einem Röntgenbild, das mit einem schwenkbar montierten Röntgengerät aufgenommen wird. Das Verfahren ist allgemein mit Bezugszeichen 100 bezeichnet.

Das Verfahren 100 umfasst einen Schritt S102 des Empfangens von Bewegungszustandsdaten, die auf einen Bewegungszustand eines Röntgengeräts hinweisen. Das Röntgengerät ist auf einem schwenkbaren Arm montiert. Der Bewegungszustand ist einem Zeitpunkt eines Aufnehmens eines Röntgenbilds mittels des Röntgengeräts zugeordnet.

Das Verfahren 100 umfasst ferner einen Schritt S104 des Bestimmens einer Position des Röntgengitters, das am Detektor des Röntgengeräts angeordnet ist. Die Position des Röntgengitters umfasst eine Position relativ zur Röntgenquelle des Röntgengeräts. Das Bestimmen S104 wird in Abhängigkeit der empfangenen S102 Bewegungszustandsdaten mittels eines (insbesondere datengetriebenen) Funktionsapproximators ausgeführt.

Das Verfahren 100 umfasst ferner einen Schritt S106 des Kompensierens (insbesondere in Echtzeit) eines Schattens des Röntgengitters auf dem aufgenommenen Röntgenbild. Das Kompensieren S106 umfasst ein Berücksichtigen der bestimmten S104 Position des Röntgengitters in dem aufgenommenen Röntgenbild.

Optional umfasst das Verfahren 100 einen Schritt S108 des Ausgebens des aufgenommenen Röntgenbilds umfassend den (insbesondere in Echtzeit) kompensierten S106 Schatten des Röntgengitters.

Fig. 2 zeigt schematisch eine beispielhafte Architektur einer Rechenvorrichtung zum Kompensieren (insbesondere in Echtzeit) eines Schattens eines Röntgenstreustrahlenabsorptionsgitters in einem Röntgenbild, das mit einem schwenkbar montierten Röntgengerät aufgenommen wird. Die Rechenvorrichtung ist allgemein mit dem Bezugszeichen 200 bezeichnet.

Die Rechenvorrichtung 200 umfasst eine Empfangsschnittstelle 202, die zum Empfangen von Bewegungszustandsdaten, die auf einen Bewegungszustand eines Röntgengeräts hinweisen, ausgebildet ist. Das Röntgengerät ist auf einem schwenkbaren Arm montiert. Der Bewegungszustand ist einem Zeitpunkt eines Aufnehmens eines Röntgenbilds mittels des Röntgengeräts zugeordnet.

Die Rechenvorrichtung 200 umfasst ferner einen (insbesondere datengetriebenen) Funktionsapproximator 204, der zum Bestimmen einer Position eines Röntgengitters, das am Detektor des Röntgengeräts angeordnet ist, ausgebildet ist. Die Position des Röntgengitters umfasst eine Position relativ zur Röntgenquelle des Röntgengeräts. Das Bestimmen wird in Abhängigkeit der empfangenen Bewegungszustandsdaten ausgeführt.

Die Rechenvorrichtung 200 umfasst des Weiteren eine Kompensiereinheit 206, die zum Kompensieren (insbesondere in Echtzeit) eines Schattens des Röntgengitters auf dem aufgenommenen Röntgenbild ausgebildet ist. Das Kompensieren umfasst ein Berücksichtigen der bestimmten Position des Röntgengitters in dem aufgenommenen Röntgenbild.

Optional umfasst die Rechenvorrichtung 200 eine Ausgabeschnittstelle 208, die zum Ausgeben des aufgenommenen Röntgenbilds umfassend den (insbesondere in Echtzeit) kompensierten Schatten des Röntgengitters ausgebildet ist.

Die Empfangsschnittstelle 202, und optional die Ausgabeschnittstelle 208, kann in einer Eingabe-Ausgabe-Schnittstelle 210 angeordnet sein.

Der Funktionsapproximator 204 und die Kompensiereinheit 206 können durch einen Prozessor 212 verkörpert sein. Der Prozessor kann beispielsweise eine oder mehrere CPUs und/oder GPUs umfassen.

Die Rechenvorrichtung 200 kann ferner eine Speichereinheit (kurz: Speicher) 214 umfassen.

Die Rechenvorrichtung 200 kann zum Ausführen des Verfahrens 100 ausgebildet sein. Beispielsweise können die Empfangsschnittstelle 202, der Funktionsapproximator 204, die Kompensiereinheit 206, und optional die Ausgabeschnittstelle 208, dazu ausgebildet sein, die Schritte S102, S104, S106, und optional S108, ausführen.

Ein System (nicht gezeigt) kann die Rechenvorrichtung 200 und einen oder mehrere Röntgengeräte, die jeweils auf einem schwenkbaren Arm montiert sind, umfassen. Beispielsweise kann die Rechenvorrichtung 200 das Verfahren 100 für eine Mehrzahl Röntgengeräten, die jeweils auf einem schwenkbaren Arm montiert sind, jeweils unabhängig (und/oder getrennt) ausführen.

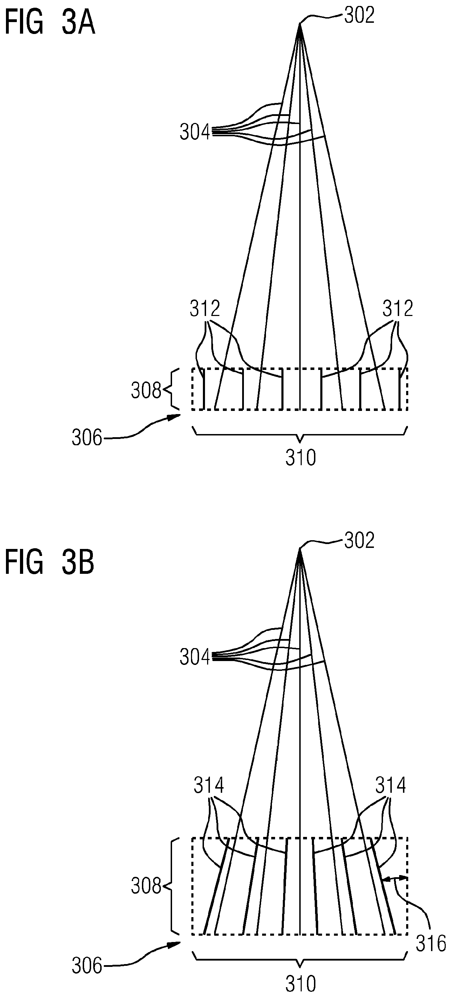

Fig. 3A zeigt ein schematisches Beispiel eines herkömmlichen Röntgengitters mit senkrechten (und/oder zueinander parallelen) Lamellen 312. Das Röntgengitter umfasst eine mit Bezugszeichen 308 bezeichnete Höhe und eine mit Bezugszeichen 310 bezeichnete Breite, oder einen Durchmesser, einer Grundfläche 306. Ausgehend von der Röntgenquelle 302 fächert der Röntgenstrahl auf, wie an Bezugszeichen 304 schematisch gezeigt.

Im Fall des herkömmlichen Röntgengitters der Fig. 3A ist der von den äußeren Lamellen verursachte Schatten unabhängig von der Position des Röntgengitters in der zur Grundfläche 306 parallelen Detektorebene.

Fig. 3B zeigt ein Beispiel eines "Smartgrids" (auch: Fokussiertes Raster). Die gleichen Größen wie in Fig. 3A sind mit identischen Bezugszeichen bezeichnet. An Bezugszeichen 316 ist beispielhaft eine maximale Winkelabweichung (z.B. 20° und/oder π/9) von einer Senkrechten einer äußeren Lamelle 314 gezeigt.

Im Fall des "Smartgrids" der Fig. 3B findet im Idealfall (insbesondere ohne ein Verschieben und/oder Auswandern des Detektors) keine Abschattung statt. Aufgrund einer Verschiebung des Detektors relativ zur Röntgenquelle 302 können unterschiedlich stark ausgeprägte (insbesondere vom Bewegungszustand des Röntgengeräts abhängige) Schatten der Lamellen 314 des Röntgengitters auftreten.

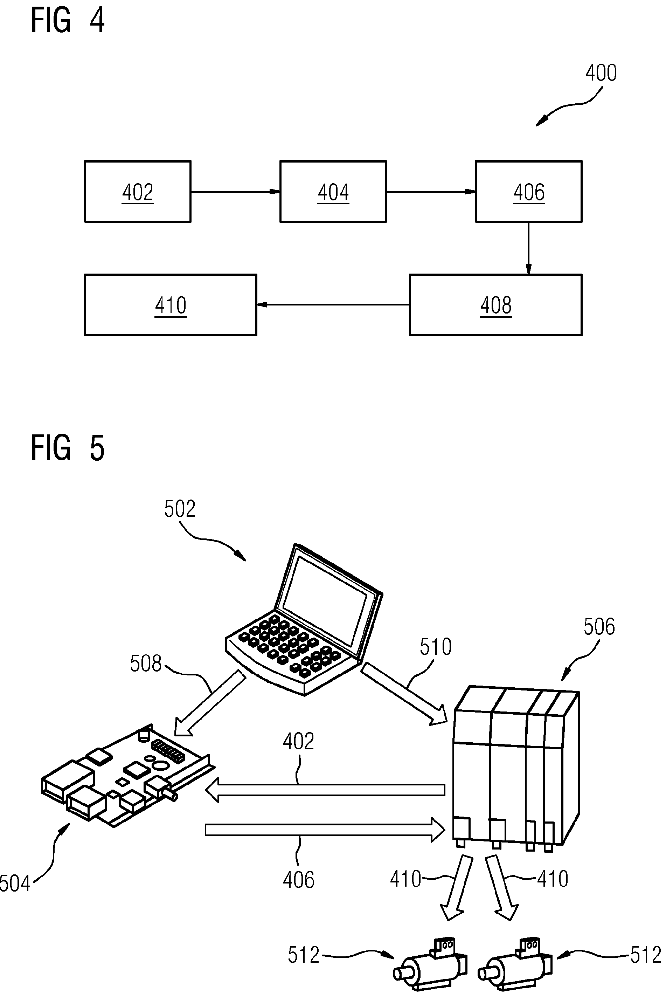

Fig. 4 zeigt ein beispielhaftes konventionelles Verfahren 400, in dem ein elastostatisches Modell der dynamischen Effekte erstellt und zur Laufzeit gerechnet wird, um die relative Verschiebung zwischen Strahler und Detektor durch Korrekturantriebe und einer kardanischen Aufhängung zu korrigieren.

An Bezugszeichen 402 wird eine Trajektorie (auch: Bewegungsablauf und/oder Bewegungszustandskurve) eines C-Bogens (als Beispiel eines schwenkbaren Arms) vorgegeben. An Bezugszeichen 404 wird ein echtzeitfähiges (z.B. elastostatisches) Modell der Mechanik des C-Bogens erstellt und/oder angewendet.

An Bezugszeichen 406 werden Verformungen des C-Bogens bestimmt (beispielsweise berechnet). An Bezugszeichen 408 wird die aus der Verformung resultierende relative Verschiebung zwischen Strahler und Detektor bestimmt. An Bezugszeichen 410 wird eine Korrektur der relativen Verschiebung durch zwei Korrekturantriebe (und/oder Kompensationsaktoren) bestimmt.

Fig. 5 zeigt Komponenten, die in dem konventionellen Verfahren 400 der Fig. 4 eingesetzt werden können. An Bezugszeichen 502 ist schematisch eine Benutzeroberfläche eines Computers (und/oder eine Rechenvorrichtung) gezeigt. An Bezugszeichen 504 ist beispielhaft eine Platine des Computers (und/oder der Rechenvorrichtung) oder eine externe Rechenvorrichtung gezeigt, auf der das echtzeitfähige (z.B. elastostatische) Modell der Mechanik ausgeführt bzw. dorthin ausgeleitet (und/oder ein Code generiert) 508 wird. Mittels des Computers (und/oder der Rechenvorrichtung 502 wird, wie an Bezugszeichen 506 angedeutet, eine Ansteuerung auf eine oder mehrere Steuereinheiten (z.B. Speicherprogrammierbare Steuerungen, SPS) 506 der Korrekturantriebe (und/oder Kompensationsaktoren) 512 projiziert.

Gemäß einer weiteren Variante (z.B. einer aktiven physikalischen Kompensation mittels Kompensationsaktorik) kann mittels Korrekturtabellen ein Verfahrbereich des C-Bogens erfasst werden, z.B. anhand von Stützstellen selektiv Abweichungen messtechnisch erfasst und zwischen den Stützpunkten interpoliert werden. Mittels der Korrekturtabellen werden nachteiligerweise nur statische Effekte erfasst, und der Einfluss von Beschleunigungskräften und Fliehkräften wird vernachlässigt werden. Weiterhin nachteilig an dieser Variante ist, dass eine Dimensionalität der Korrekturtabellen den handhabbaren Bereich (z.B. der Verschiebungen der Detektorebene) auf wenige Dimensionen beschränkt. Ferner ist nachteiligerweise ein großer messtechnischer Aufwand erforderlich. Ebenfalls nachteiligerweise wird der Bedarf an Aktorik zur aktiven Kompensation durch diese Variante nicht behoben.

Die Dimensionalität der Korrekturtabelle kann umfassen, dass von einem Raum bewegter Achsen ausgegangen wird, der n-dimensional (z.B. mit n=5) sein kann, und/oder wenn Geschwindigkeiten dazu genommen werden, 2n-dimensional (z.B. mit 2n=10) sein kann. n≥M kann eine Anzahl (z.B. über Nebenbedingungen teilweise abhängiger für n>M) Freiheitsgrade (und/oder verallgemeinerte Koordinaten) bei M unabhängigen Freiheitsgraden umfassen. Ein Ermitteln und Anlegen der Korrekturtabelle für alle Kombinationen entsprechender Korrekturdaten kann (z.B. praktisch) nicht handhabbar sein. Mittels der erfindungsgemäßen Technik (z.B. umfassend das Verfahren 100 und/oder die Rechenvorrichtung 200) kann der Aufwand reduziert werden auf die 2D (zweidimensionale) Detektorebene, insbesondere mit einem Gitter (auch: Grid) von Messpunkten entlang der 2D Detektorebene.

Alternativ oder ergänzend kann durch konstruktionstechnische Maßnahmen versucht werden, die Verschiebungen aufgrund der Deformationen zu minimieren, beispielsweise durch weitere Versteifung des Arms, insbesondere des C-Bogens. Dies hat jedoch im Allgemeinen den Nachteil des vermehrten Materialbedarfs zur Herstellung und eines höheren Gesamtgewichts des Röntgengeräts und führt beispielsweise trotzdem zu nicht ausreichend geringen Abweichungen.

Mittels der erfindungsgemäßen Technik (insbesondere umfassend das Verfahren 100 und/oder die Rechenvorrichtung 200) können eine zusätzlich benötigte Aktorik zur aktiven Kompensation der Gitterverschiebung am Detektor und/oder konstruktionstechnische Maßnahmen zur Versteifung des C-Bogens (beispielsweise gänzlich) vermieden werden.

Die aufgrund der Verschiebung eintretende Beeinträchtigung wird in der (beispielsweise zunächst) Bildgebung als gegeben in Kauf genommen. Wird der Arm (z.B. C-Bogen) in eine stark deformierende Stellung gebracht (z.B. laterale Angulation), so wird das Röntgengitter im Röntgenbild (z.B. deutlich) sichtbar werden, obwohl das Gitter kalibriert wurde.

Das Problem des im aufgenommenen Röntgenbild (z.B. deutlich) sichtbaren Röntgengitters kann grundsätzlich dadurch behoben werden, dass in der neuen Position die Bildgebung wieder kalibriert wird. In der Folge kann eine Vielzahl von (z.B. statischen) Kalibrierungspunkten anfahren werden. Alternativ oder ergänzend kann in einer dynamischen Situation (z.B. bei einer schnellen C-Bogen-Fahrt) kein Kalibrierungspunkt mehr vorhanden sein.

Mittels der erfindungsgemäßen Technik (insbesondere umfassend das Verfahren 100 und/oder die Rechenvorrichtung 200) wird zunächst vom hochdimensionalen Raum der Gelenkkonfiguration in die für die Bildgebung relevante Ebene reduziert (und/oder gerechnet), in der die Verschiebung des Röntgengitters letztlich zu betrachten ist.

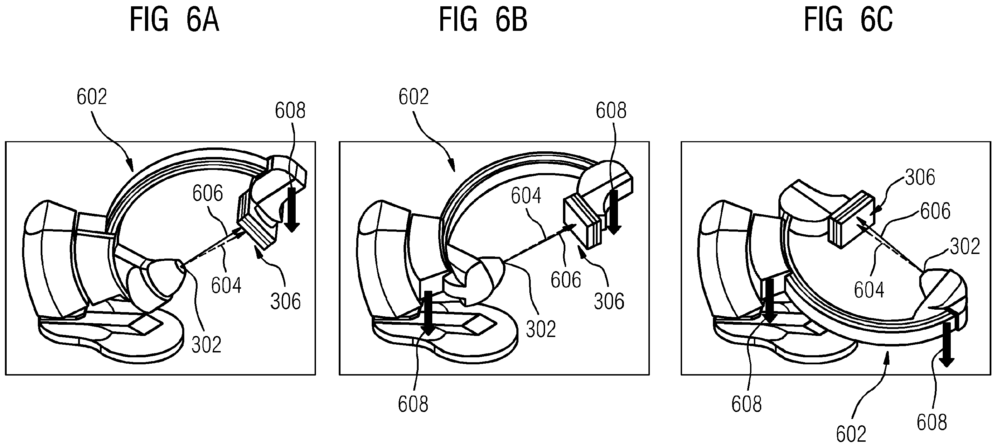

Fig. 6A, 6B und 6C zeigen beispielhaft einen C-Bogen 602 in unterschiedlichen Stellungen. Der C-Bogen 602 umfasst eine Röntgenquelle 302 und am gegenüberliegenden Ende einen Detektor mit Röntgengitter auf der Detektorebene 306. Auf die Enden des C-Bogens 602 wirkt die beispielhaft an Bezugszeichen 608 eingezeichnete Schwerkraft unterschiedlich je nach Stellung des C-Bogens. Aufgrund der unterschiedlichen, zur Laufzeit wirkenden physikalischen Kräfte 608 wird die an Bezugszeichen 604 gezeigte unverschobene (und/oder ideale, beispielsweise durch aktive Kompensation mittels einer Kompensationsaktorik, erreichte) Richtung des Röntgenstrahls in verschiedene Richtungen und/oder mit unterschiedlicher Ausprägung verschoben, wie jeweils an Bezugszeichen 606 gezeigt.

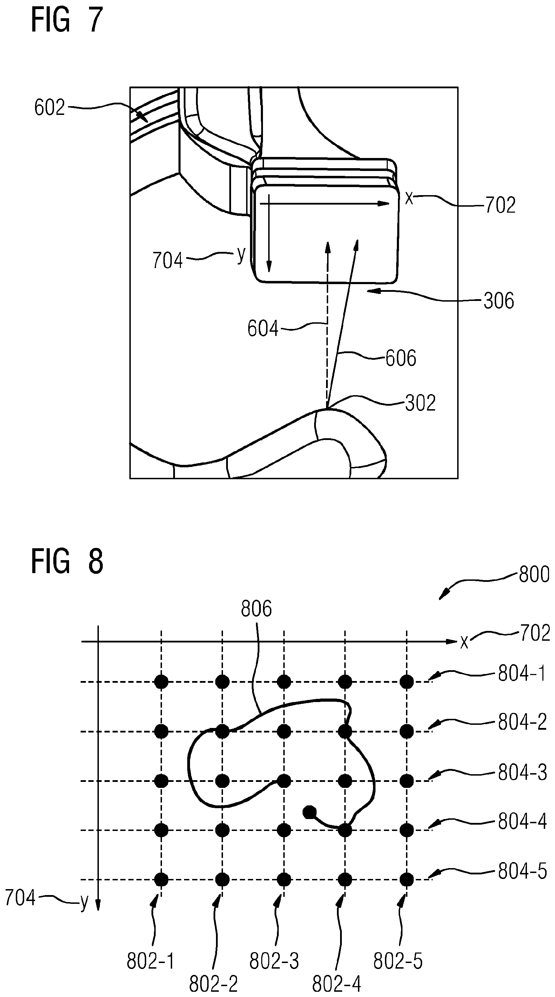

Fig. 7 zeit die Grundfläche 306 des Röntgengitters, die entlang der Detektorebene liegt, sowie die Röntgenquelle 302 aus einer weiteren Perspektive. Wie in Fig. 7 gezeigt, kann die Detektorebene durch 2D Koordinaten (x,y) an Bezugszeichen 702 und 704 parametrisiert (und/oder aufgespannt) werden.

In dem schematischen Ausführungsbeispiel der Fig. 7 ist der Ursprung (0,0) des (x,y)-Koordinatensystems an einer Ecke des Detektors eingezeichnet. In anderen Ausführungsbeispielen kann der Ursprung (0,0) des (x,y)-Koordinatensystems in der Mitte der Detektorebene, und/oder an der Stelle der Ruheposition des Fokuspunkts des Röntgenstrahls liegen.

Der Schritt des Reduzierens vom hochdimensionalen (z.B. M- oder 2M-dimensionalen, wobei M eine Anzahl unabhängiger, insbesondere translatorischer und rotatorischer, Freiheitsgrade umfasst) Raum der Gelenkkonfiguration in die für die Bildgebung relevante Detektorebene (auch: 2D-Projektion) und/oder Grundfläche des Röntgengitters kann anhand eines vereinfachten Modells erfolgen

Vorteilhafterweise kann mittels der erfindungsgemäßen Technik der Schritt des Reduzierens vom hochdimensionalen Raum der Gelenkkonfiguration in die für die Bildgebung relevante Ebene unter Verwendung des Funktionsapproximators, insbesondere ausgebildet als Maschinelles Lernen (fachsprachlich: Machine Learning, ML) und/oder als neuronales Netzwerk (NN), zur Laufzeit mit nur sehr geringer Rechenzeit ausgeführt werden.

Zum Training des NN können Daten erhoben werden, die für die positionsabhängige und/oder geschwindigkeitsabhängige Verformung des C-Bogens charakteristisch sind. Die Datensammlung kann durch hochgenaue Simulationen unter Berücksichtigung der dynamisch auftretenden Biegeeffekte, Labormessungen, oder einer Kombination daraus erfolgen. Beispielsweise kann durch simulierte Daten ein zunächst vom genauen Typ des Geräts abhängiges NN (und/oder Modell) trainiert werden, welches durch Messungen am realen Gerät noch verfeinert wird.

Das Resultat des Trainingsprozesses kann ein Modell f sein, welches die direkte Abbildung des kinetischen Gerätezustands (und/oder Bewegungszustandsdaten, insbesondere Position und/oder Geschwindigkeit der Achsen und/oder Gelenke) auf die für die Verschiebung relevante (x,y)-Ebene abbildet: (x,y) = f (Bewegungszustandsdaten, insbesondere der Achsen).

Je nach Stärke der vorherrschenden Effekte kann ein Feedforward NN (FNN) mit er geeigneter Zeiteinbettung (fachsprachlich: Time Embedding) der Eingangsdaten und/oder auch ein rekurrentes NN (RNN) verwendet werden.

Die Anzahl der Möglichkeiten, welche anschließend durch Substraktionsrechnung im Vergleich zum kalibrierten Bild gerechnet werden müssen, ist durch den Einsatz des Funktionsapproximaters, insbesondere des ML und/oder NN, erheblich reduziert und kann z.B. durch ein Gridding in der Detektorebene dargestellt werden.

Eine Bildkalibrierung kann anhand einer vorbestimmten (und/oder begrenzten) Anzahl von Verschiebungsvektoren, beispielsweise 5x5 = 25, erfolgen. Ein (z.B. jeder) Verschiebungsvektor kann anhand der Bewegungszustände der Gelenke (insbesondere umfassend Winkel und/oder Winkelgeschwindigkeiten) sowie Deformationen aufgrund von Kräften (z.B. Schwerkraft, Corioliskraft, und/oder Zentrifugalkraft) bestimmt werden.

In Fig. 8 ist beispielhaft ein 5x5 Gitter 800 dargestellt, welches zumindest einen Teil der Detektorfläche in 16 Felder unterteilt. Die 16 Felder sind in Fig. 8 zwischen den horizontalen Richtungen 804-J (J=1,..,5) und den vertikalen Richtungen 802-I (I=1,..,5) angeordnet. Die Kalibrierpunkte können jeweils an einem Schnittpunkt einer vertikalen und einer horizontalen Richtung angeordnet sein und/oder mit (802-I;804-J) bezeichnet werden.

Ein Abstand der Kalibrierpunkte kann unabhängig von (insbesondere kleiner als) einer Gitterkonstanten (und/oder einem Abstand auf der Grundfläche paralleler Lamellen) des Röntgengitters sein. Beispielsweise können die in Fig. 8 gezeigten Kalibrierpunkte innerhalb einer Wabe oder eines Rasterabschnitts liegen.

Gemäß dem in Fig. 8 schematisch gezeigten Ausführungsbeispiel werden 25 Kalibrierbilder erstellt, die als Grundlage für die Bildrekonstruktion unter beliebigen Verschiebungen dient.

Der beispielhaft dargestellte Pfad 806 des Fokalpunkts, welcher aufgrund der Deformation unter der Bewegung des C-Bogens auswandert, ist mittels des Funktionsapproximators, insbesondere als NN ausgebildet, zur Laufzeit berechenbar.

Zur Kompensation der entstehenden Gitterschatten im Röntgenbild kann in einem weiteren Ausführungsbeispiel ein Nächster-Nachbar (fachsprachlich: Nearest-Neighbor) Ansatz gewählt werden, um den nächst-passenden Kalibrierpunkt zu wählen, und/oder um aus den vorhandenen Kalibrierpunkten gewichtet zu interpolieren und somit auch an jeder Stelle des Pfades 806 eine quasi-Kalibrierung und damit angemessene Bildqualität zu gewährleisten.

Gemäß einem weiteren Ausführungsbeispiel kann ein berechneter Verschiebungsvektor (insbesondere in der (x,y)- und/oder Detektorebene) nicht zur Interpolation zwischen vorab aufgenommenen Kalibrierpunkten verwendet werden, sondern um eine direkte (insbesondere AI-basierte) Bildrekonstruktion zu unterstützen, welche die zwangsläufig auftretenden, unerwünschten Artefakte aus dem aufgenommenen Bild herausrechnet (z.B. mittels eines tiefen CNN). In diesem Ausführungsbeispiel kann die korrekte Verschiebung eine sehr wertvolle Erweiterung eines Feature-Spaces darstellen und damit als Grundlage zur Entwicklung eines AI-gestützten Rekonstruktionsalgorithmus zur Bildrekonstruktion dienen.

Herkömmlicherweise kann ein Kompensieren durch Bildkalibrierung nicht ausführbar sein aufgrund der hohen Dimensionalität (beispielsweise des Bewegungszustandsraums) und/oder aufgrund der großen Anzahl zu betrachtender kombinatorischer Möglichkeiten (beispielsweise von Bewegungen entlang unterschiedlichen Freiheitsgraden).

Die erfindungsgemäße Technik (insbesondere umfassend das Verfahren 100 und/oder die Rechenvorrichtung 200) kann eine Kombination aus sehr genauen (fachsprachlich: High-Fidelity) Simulationen und/oder Messungen mit (z.B. ML-) Regression (insbesondere unter Verwendung des Funktionsapproximators, insbesondere ausgebildet als NN, verwenden.

Im Rahmen der erfindungsgemäßen Technik (insbesondere umfassend das Verfahren 100 und/oder die Rechenvorrichtung 200) ist ein Kompensieren durch Bildrekonstruktion möglich, indem nur eine begrenzte Anzahl von Kalibrierpunkten in der (x,y)-Ebene des Detektors verwendet wird und dann interpoliert wird, beispielsweise mittels einer gewichteten Nächsten-Nachbar-Methode.

Das in Fig. 8 beispielhaft gezeigte Kalibrier-"Raster" 800 mit notwendigen Kalibrierpunkten und/oder Korrekturtabellen muss sich nicht über die komplette (x,y)-Ebene des Detektors erstrecken. Die Koordinatensysteme der Fig. 7 und 8 können somit nur für einen kleinen Bereich (beispielsweise im Zentrum) der Detektorebene verwendet werden. Die schematischen Darstellungen in Fig. 7 und 8 sind, ebenso wie alle weiteren schematischen Darstellungen, nicht als maßstabsgetreu zu verstehen.

Beispielsweise können Auslenkungen (insbesondere aufgrund von dynamischen Effekten und/oder ja nach Stellung unterschiedlich wirkender Schwerkraft) um ca. 5mm in x-Richtung und ca. 7mm in y-Richtung gemessen werden.

Wie stark der Fokuspunkt des Röntgenstrahls auswandert, hängt von verschiedenen Parametern ab (z.B. der Steifigkeit des C-Bogens, und/oder einer Verfahrgeschwindigkeit). Der relevante Bereich der Verschiebungen in der Detektorebene kann sich beispielsweise nur in einem Bereich von 2,5cm×2,5cm um den Mittelpunkt der Detektorfläche erstrecken.

Die erfindungsgemäße Technik (insbesondere umfassend das Verfahren 100 und/oder die Rechenvorrichtung 200) unterscheidet sich von herkömmlichen Kompensationstechniken dadurch, dass keine (z.B. zusätzliche, und/oder Kompensations-) Aktorik notwendig ist, um das Röntgengitter mechanisch der Verschiebung aufgrund der auftretenden Deformationen nachzuführen. Somit kann ein "Smartgrid" zum Einsatz kommen, ohne dass der Verbau zusätzlicher motorisierter Achsen notwendig wird. Folglich lassen sich erhebliche Einsparungen in Material und technischer Baukomplexität des Röntgengeräts erreichen, indem das Problem auf die Berechnung des Verschiebepunkte und einiger Kalibrationsaufnahmen reduziert wird.

Ein weiterer Vorteil der erfindungsgemäßen Technik liegt darin, dass die Berechnung des Verschiebungspunktes und damit auch der anzuwenden Bildkalibration zur Laufzeit mit sehr geringer Latenz möglich ist. Dies steht im Gegensatz zu einem mittels (z.B. zusätzlicher, und/oder Kompensations-) Aktorik herkömmlich realisierbaren Dynamikumfang und/oder der Trägheit der mechanischen Verstellung.

Die Erfindung kann beispielsweise sichtbar gemacht werden, indem in einem Angiographiegerät ein "Super Grid" zum Einsatz kommt, dieses jedoch nicht durch Aktorik nachgeführt und/oder feinjustiert wird und stattdessen ein Anfahren diverser Kalibrierungspunkte sichtbar ist.

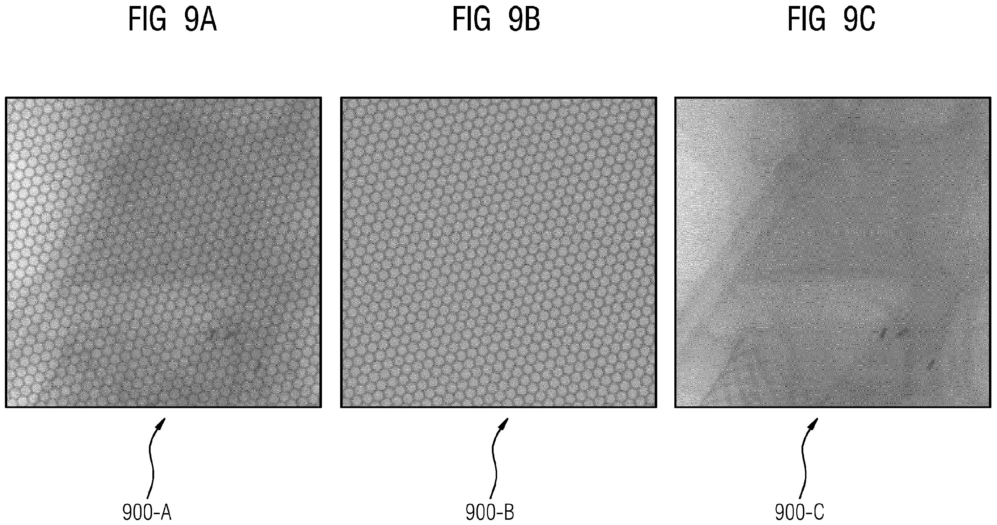

Fig. 9A, 9B und 9C zeigen beispielhaft eine Subtraktion eines Kalibrierbilds. Von dem mit Röntgengitter aufgenommenen Röntgenbild 900-A eines Patienten in Fig. 9A wird ein Kalibrierbild des Röntgengitters 900-B in Fig. 9B abgezogen (und/oder subtrahiert), so dass das in Fig. 9C gezeigte Subtraktionsbild 900-C entsteht, welches Strukturen des Patienten zeigt.

In einer beispielhaften Ausführungsform beträgt ein Abstand vom Ursprung des Röntgenstrahls zu Detektor ca. 1,2m, die Abmaße des Detektorgitter liegen zwischen 20cm×20cm und 45cm×45cm (wobei der Detektor auch rund sein kann mit einem Durchmesser von 20cm bis 45cm) und/oder die Gitterhöhe eines "herkömmlichen Röntgenrasters" (insbesondere mit senkrecht ausgerichteten Lamellen ) kann ca. 5mm betragen, während eine Gitterhöhe eines Fokussierten Rasters (und/oder "Smartgrid", insbesondere mit in Richtung der Röntgenquelle fokussiert ausgerichteten Lamellen, auch als Hochaspektröntgengitter bezeichnet) bis zu 2cm betragen kann. Die äußersten Strahlen und/oder Lamellen des Fokussierten Rasters können einen Winkel bis zu 20° (und/oder π/9) zu einer Senkrechten der Detektoreben aufweisen.

Das Röntgengitter kann fest am Detektor angebracht sein. Die Vorteile durch das Hochaspektröntgengitter sind grundsätzlich bei allen diagnostischen röntgenstrahlungsbasierten Bildgebungssystemen erstrebenswert.

Die erfindungsgemäße Technik mittels eines NN (als Ausführungsbeispiel eines Funktionsapproximators) realisiert sein und einen n-dimensionalen Raum der C-Bogen-Konfigurationen auf den 2D Raum der Detektorebene abbilden. Die Dimension n kann größer gleich der Anzahl M unabhängiger Freiheitsgrade des C-Bogens sein. Beispielsweise kann der n-dimensionale Raum auch redundante Freiheitsgrade (z.B. mit Nebenbedingungen) umfassen.

Für die Verschlechterung der Bildqualität kann insbesondere die Verschiebung des Röntgenstrahls auf der Detektoroberfläche maßgeblich und/oder ausschlaggebend sein (insbesondere in 2D). Aufgrund welcher physikalischer Effekte die Verschiebung zustande kommt, kann für die erfindungsgemäße Technik irrelevant sein.

Es können folgende Größen für die nachfolgenden Ausführungsbeispiele definiert werden:
n E N: Anzahl der beweglichen Freiheitsgrade des C-Bogens;
$q ∈ {ℝ}^{n}$: Gelenkwinkel der Achsen und/oder generalisierte Koordinaten;
$\dot{q} ∈ {ℝ}^{n}$: Geschwindigkeiten (und/oder zeitliche Ableitungen der Gelenktwinkel) der Achsen und/oder generalisierte Geschwindigkeiten;
${q}^{¨} ∈ {ℝ}^{n}$: Beschleunigungen der Achsen und/oder generalisierte Beschleunigungen;
$\left(x, y\right) ∈ {ℝ}^{2}$ : Koordinatenrichtungen in der Detektorebene; und
$\overline{x} = \left[\begin{matrix}q \\ \dot{q}\end{matrix}\right] ∈ {ℝ}^{2 n}$: (insbesondere Dynamischer Bewegungs-) Zustand des Systems.

Je nach (insbesondere dynamischem Bewegungs-) Zustand ***x̅*** können verschiedene (z.B. statische und/oder dynamische) Kräfte auf das Bildgebungssystem wirken.

Unter den statisch wirkenden Kräften (insbesondere für ***q̇*** = 0) sind vor allem die positionsabhängig wirkenden Gravitationskräfte ***G*(*q*)** relevant. Die Gravitationskräfte wirken über das ganze Röntgengerät hinweg und verursachen, aufgrund der limitierten Steifigkeit des C-Bogens, unterschiedliche Auslenkungen am Strahler und am Detektor. Je nach Pose des C-Bogens hat dies verschiedene Auswanderungen des Röntgenstrahls in der **(x,y)**-Ebene des Gitters zur Folge, wie in Fig. 6A, 6B und 6C beispielhaft gezeigt.

Analog verhält es sich mit den nur bei Bewegung bzw. Beschleunigung (beispielsweise für ***q̇*** ≠0) auftretenden Kräften, z.B. Corioliskräften und/oder Zentrifugalkräften, die typischerweise als ***C*(*q, q̇)q̇*** zusammengefasst werden. Aufgrund dieser Trägheitskräfte wird ebenso eine Verschiebung in der **(x,y)-**Ebene auftreten.

***G(q)*** und ***C*(*q, q̇)q̇*** können eine ***n-*** bzw. **2*n***-dimensionale Abhängigkeit haben. Bei einem Standard-C-Bogen mit z.B. n = 5 Freiheitsgraden entstehen somit, wenn jeder Freiheitsgrad nur in zehn (10) diskrete Werte unterteilt wird, schon 10⁵ bzw. 10¹⁰ (für ***G*(*q*)** bzw. ***C*(*q, q̇*)*q̇*)** mögliche Kombinationen, für die Kalibrationsdaten zur Kompensation der zugehörigen Verschiebung mittels der Bildrechnung anfertigt werden müssen. Dies ist nicht machbar.

Mit der erfindungsgemäßen Technik sind in der **(x,y)**-Ebene beispielsweise nur 5×5 = 25, wie in Fig. 8 exemplarisch gezeigt, 36 oder 49 Kalibrationsmessungen nötig, je nach gewähltem Raster und der erforderlichen Genauigkeit. Eine geeignete Anzahl Kalibrationsmessungen kann beispielsweise experimentell ermittelt werden.

Mittels der erfindungsgemäßen Technik kann eine zur Laufzeit auswertbare Vorschrift für die Berechnung $\left(x, y\right) = f \left(\overline{x}\right)$ (1) ermittelt werden, um zu jedem Bewegungszustand ***x̅*** des C-Bogens die erwartete Auswanderung ***(x,y)*** zu bestimmen.

Die technische Lösung, die sich für das Ermitteln der Vorschrift in Gleichung (1) anbietet, besteht in der Nutzung eines datengetriebenen Funktionsapproximators, insbesondere eines NN. Der (insbesondere zeit-)kontinuierliche Verlauf der physikalischen Größen, insbesondere ***q*(*t*), *q̇*(*t*),** wird zunächst in Form eines zu äquidistanten Zeitpunkten ***t*₀, *t*₁, *. ..*** abgetasteten, diskretisierten Signals **{*q*[*t*]} = {*q*(*t*₀), *q*(*t*₁),*...*}** bzw. **{*q̇*[*t*]} = {*q̇*(*t₀*), *q̇*(*t*₁),...}** dargestellt.

Im ersten Schritt werden (insbesondere geeignete) Trainingsdaten für den Funktionsapproximator erhoben werden. Da es sich insbesondere um Überwachtes Lernen handelt, werden Datenpaare benötigt, die beide Seiten von Gleichung (1) charakteristisch miteinander in Verbindung bringen. Z.B. für die positionsabhängige und geschwindigkeitsabhängige Verformung des C-Bogens müssen entsprechende Werte der Auslenkungen **(x,y)** am Detektor ermittelt werden.

Für die Zuordnung von Werten der Auslenkungen **(x,y)** zu positionsabhängigen und geschwindigkeitsabhängigen Verformungen gibt es zwei grundsätzliche Herangehensweisen.

In der ersten Herangehensweise werden die Zuordnungen durch Simulation bestimmt. Hierfür wird ein hochgenaues Simulationsmodell des Gerätes erstellt, welches die Verformung unter Einfluss der Bewegungszustandsgrößen berechnen kann, z.B. mittels numerischen Finite Element Solvern über ein elastisches Mehrkörperdynamikmodell. Eine große Zahl von zufälligen Bewegungsabläufen **{*q*[*t*]}** kann bestimmt (und/oder auf das Modell aufgeprägt) werden. Durch die in der Simulation auftretenden Verformungen werden die Zeitverläufe der zu erwartenden Auswanderung **({*x*[*t*]}*,* {*y*[*t*]})** in der Detektorebene bestimmt. Die mittels der ersten Herangehensweise bestimmte Menge der erhaltenen Datenpunkte **{((*q*[*t₀*],*q̇*[*t*₀])*,*(*x*[*t₀*], *y*[*t₀*])), ((*q*[*t*₁]*, q̇*[*t₁*]), (*xt*₁, *y*[*t*₁])),...}** kann als ***Uₛᵢₘ*** bezeichnet werden.

In der zweiten Herangehensweise erfolgt die Bestimmung in einem experimentellen Setup (und/oder mittels Labormessungen). Hierfür wird zunächst eine Möglichkeit geschaffen werden, die Auswanderung des Strahls experimentell nachzustellen und zu messen, beispielsweise durch eine Versuchsanordnung mit einem Lasertracker. Anschließend werden in einem Versuch eine große Zahl zufälliger Bewegungen durchfahren. Die Menge der mittels der zweiten Herangehensweise aufgezeichneten Datenpunkte **{((*q*[*t*₀]*,q̇*[*t₀*]), (*x*[*t₀*], *y*[*t₀*])), ((*q*[*t*₁]*, q̇*[*t₁*]), (*xt*₁, *y*[*t*₁])),...}** kann als ***Uₑₓₚ*** bezeichnet werden.

Beide Herangehensweisen haben prinzipbedingte Vorteile und Nachteile. Simulativ können leichter große Datenmengen erhalten werden, allerdings ist die Genauigkeit durch die Modellgüte bestimmt. Experimentell ist der messtechnische Aufwand größer. Ein Vorteil der experimentellen Herangehensweise besteht darin, dass der reale Versuch auch Effekte beinhalten kann, welche in der Simulation nicht (oder nicht ausreichend) modelliert sind, beispielsweise aufgrund fertigungsbedingter Toleranzen.

Die beiden Herangehensweisen sind miteinander kombinierbar. Beispielsweise kann ein erster Teil der Trainingsdaten als simulierte Trainingsdaten mittels der ersten Herangehensweise erhalten werden und ein zweiter Teil der Trainingsdaten als reale Trainingsdaten mittels der zweiten Herangehensweise erhalten werden.

Eine erste bespielhafte Architektur des NN und der zugehörige Aufbau der Merkmalsvektoren kann ein Feedforward Neural Network (FNN) umfassen.

Ein FNN kann bei hinreichender Komplexität (z.B. mit mehreren Schichten als Multilayer Perceptron Network) als universeller Funktionsapproximator eingesetzt werden und daher die gewünschte Abbildung in Gleichung (1) grundsätzlich mittels einem tiefem Lernverfahren (fachsprachlich: Deep Learning, DL) erlernen.

Das FNN kann gemäß einem ersten Ausführungsbeispiel wie folgt aufgebaut sein. Eine Eingangsschicht (fachsprachlich: Input Layer) kann **2*n*** Knoten (und/oder Neuronen) umfassen, insbesondere um den kompletten Zustand ***x̅*** als Eingang zu verwenden.

Es können ein bis viele verborgene Schichten (fachsprachlich: Hidden Layers) in dem FNN verwendet werden. Die Anzahl und Eigenart, insbesondere der Perzeptrons, kann experimentell optimiert werden. Eine Übertragungsfunktion der einzelnen Neuronen kann auf herkömmliche Weise gewählt werden und z.B. eine lineare Funktion, eine Sigmoid-Funktion und/oder eine logistische Funktion umfassen. Grundsätzlich kann angestrebt werden, die Anzahl der Layer und Neuronen so gering wie möglich (und damit insbesondere die Komplexität so einfach wie möglich), doch so hoch wie nötig zu wählen.

Eine Ausgangsschicht (fachsprachlich: Output Layer) kann zwei Neuronen umfassen, da das gewünschte Signal (und/oder die Verschiebung in der Detektorebene) zweidimensional ist.

Das NN, insbesondere das FNN und/oder ein RNN, kann durch Standard-Verfahren wie Backpropagation trainiert werden, indem die Datenpunkte aus ***Uₛᵢₘ*** und/oder ***Uₑₓₚ*** verwendet werden. Herkömmliche Vorgehensweisen, um die Trainingsperformance zu verbessern, z.B. eine Normalisierung der Ausgangsdaten auf den Bereich [-1, 1] sowie anschließende Rückskalierung, können angewendet werden.

Nach dem Training bildet das NN, insbesondere das FNN, einen mathematischen und/oder näherungsweisen (auch: approximativen) Zusammenhang der Datenpunkte wie folgt ab: $\left(x\left[t\right],y\left[t\right]\right) = \hat{f} \left(\left(q\left[t\right],\dot{q}\left[t\right]\right)\right) .$ (2)

Eine wichtige Eigenschaft des FNN besteht darin, dass die Beziehung in Gleichung (2) nicht nur für die zum Training verwendeten Daten ***U*** gilt, sondern auch für andere, nicht trainierte Dateneingangspunkte **(*q, q̇*).**

Wie beim Training von NNs herkömmlicherweise üblich, wird die Gültigkeit der Beziehung in Gleichung (2) während des Trainingsprozesses durch angemessene Verfahren sichergestellt, beispielsweise durch Vorhalten eines Teils der gesammelten Daten ***U*** und Vergleichsprüfung (fachsprachlich: Cross Validation). Es kann üblicherweise davon ausgehen werden, dass die Abbildung ***f̂*** aus Gleichung (2) den Zusammenhang ***f̂*** aus Gleichung (1) hinreichend genau approximiert.

Das fertig trainierte NN, insbesondere das FNN, ermöglicht es, zur Laufzeit des Röntgengeräts zu jeder Solltrajektorie ***q_{d}*(*t*)** den Verlauf der zu erwartenden Auswanderung (*x_{q_{d}}*(*t*)**,** *y_{q_{d}}*(*t*)) zu berechnen, indem für alle diskreten Zeitpunkte ***t*** die Gleichung (2) ausgewertet wird. Der Vorgang kann als Inferenz mittels NN und/oder Prädiktion bezeichnet werden.

Ein Verlauf der Auswanderung ist beispielhaft in Fig. 8 an Bezugszeichen 806 gezeigt.

Alternativ oder ergänzend kann, da die Inferenz mit dem NN, insbesondere dem FNN, als Funktionsapproximator nur wenig Rechenzeit erfordert, in jedem Zeitschritt ***t*** zur Laufzeit die Vorschrift der Gleichung (2) mit den Ist-Daten des Röntgengeräts bestimmt werden. Die Variante birgt den Nachteil, dass nicht nur die Position ***q*(*t*),** sondern auch die Geschwindigkeit ***q̇*(t)** bekannt sein muss, sei es durch Messung oder Schätzung.

Eine alternative oder ergänzende Variante zum Aufbau des FNNs besteht darin, nur Positionsdaten ***q*** zu verwenden und diese in ein höherdimensionales Eingangsdatum zu überführen. Beim der sogenannten Zeiteinbettung (fachsprachlich: Time Embedding) kann ein FNN trainiert werden, das die Abbildung $\left(x\left[t\right],y\left[t\right]\right) = {f}^{˜} \left({q}_{te}\left[t\right]\right)$ (3) approximiert. Der Vektor ***qₜₑ*** durch Zeiteinbettung bis zur Ordnung ***T*** gebildet, z.B. werden sukzessive Elemente des Signals ***(q[t])*** in einen Vektor gestapelt: $\left({q}_{te}\left[t\right]\right) = \left(q\left[t\right],q\left[t-1\right],q\left[t-2\right],…,q\left[t-T\right]\right) .$ (4)

Zeiteinbettung kann ein herkömmliches Verfahren in der Zeitreihenprädiktion umfassen und/oder im Zusammenhang mit einem NN, insbesondere einem FNN, angewendet werden.

Gemäß einem zweiten Ausführungsbeispiel kann der Aufbau des FNN eine Eingangsschicht (und/oder Input Laye) umfassend ***T · n*** Knoten umfassen, da der Vektor aus Gleichung (4) als Eingangsdatum verwendet wird.

Die verborgenen Schichten (und/oder Hidden Layers) und die Ausgabeschicht (und/oder Output Layer) können wie im Kontext des ersten Ausführungsbeispiel beschrieben ausgebildet sein.

Das zweite Ausführungsbeispiel hat den Vorteil, dass die für das Röntgengerät (insbesondere sowieso) gemessenen Positionsdaten ausreichend sind, um die Inferenz mit dem NN, insbesondere dem FNN, zu berechnen. Je nach erforderlicher Einbettungstiefe ***T*,** die durch Ausprobieren (fachsprachlich: Trialand-Error) ermitteln werden kann, wird so gering wie nötig gehalten, zieht dies jedoch eine erheblich vergrößerte Anzahl von zu lernenden Parametern nach sich. Der Trainingsprozess zur Entwicklungszeit des Funktionsapproximators (und/oder des Systems umfassend das Röntgengerät und die Rechenvorrichtung) wird also etwas aufwendiger als für das erste Ausführungsbeispiel.

Ein drittes Ausführungsbeispiel, das mit den vorhergehenden Ausführungsbeispielen kombinierbar sein kann, kann ein RNN umfassen.

Sollten in der Anwendung die dynamischen Effekte die auftretenden Durchbiegungseffekte und damit die Abschattung (z.B. besonders) dominieren, kann es vorteilhaft sein, anstelle des FNNs (und/oder zusätzlich) ein RNN zu verwenden. Bei einem RNN sind die einzelnen künstlichen Neuronen durch Rückführungen untereinander verbunden. Somit ist das RNN selbst fähig, Bewegungszustandsdaten zu speichern, insbesondere wie sie für dynamische Vorhersagen vonnöten sind. Ein Zeiteinbettung ist in diesem Falle nicht notwendig.

Nachteilig an einem RNN ist, dass die Lernverfahren für RNNs in der Regel langsamer konvergieren als im Falle eines FNN und/oder die Trainingsverfahren herkömmlicherweise aufwändiger sind. Eine bekannte Architektur der Klasse der RNN, die hier eine gewisse Abhilfe schaffen kann, ist das sogenannte long short-term memory network (LSTM).

RNNs kommen herkömmlicherweise hauptsächlich in Anwendungen wie Spracherkennung, Sprachsynthese und/oder automatischer Bildbeschreibung zum Einsatz. Grundsätzlich könnten RNNs jedoch auch zum Erlernen der Gleichung (1) verwendet werden.

Das Trainieren des Funktionsapproximators, insbesondere eines NN, dient dazu, zur Laufzeit schnell und genau das gerade (insbesondere erforderliche) beste Kalibrierbild auszuwählen (auch: herauszusuchen und/oder zu berechnen), um erfindungsgemäß zumindest die aufgrund der Verschiebung zwischen Röntgenquelle und Detektor auftretenden Bildartefakte mittels Kalibration der Bildgebung zu kompensieren.

Eine Auswahl des nächsten Kalibrierbilds kann beispielsweise mittels eines Nächsten-Nachbar (fachsprachlich: Nearest-Neighbor) Ansatz vorgenommen werden. Dies ist keine Heuristik zur Klassifikation, wie z.B. k-NN.

Der Nächste-Nachbar-Ansatz kann umfassen, dass zu jedem Zeitpunkt ***t*** derjenige Kalibrationspunkt aus dem Gitter (beispielsweise dem Gitter in Fig. 8) gewählt wird, welcher den geringsten Abstand zur geschätzten Auswanderung **(*x(t),y(t))*** aufweist. Beispielsweise können mit ***c_{G,1}, x_{G,2}*,*...*5** sowie ***y_{G,1}, y_{G,2},...,t*_{*G,*5}** die Koordinaten der Kalibrierbilder bezeichnet werden, welche an den entsprechenden Stellen des Rasters (z.B. (802-I;804-J) mit I,J=1,..,5) aufgezeichnet wurden. Beispielsweise kann das Koordinatenpaar **(*x*_{*G,*1}*, y_{G,1}*)** dem Gitterpunkt (802,1;804-1) entsprechen. Die Vorschrift zur Auswahl der Kalibration lautet dann, falls bspw. Gleichung (3) zum Einsatz kommt: $\left({i}_{best},\right. \left({j}_{best}\right) = arg {min}_{\left(i, j\right)} \left‖\hat{f}\left(q\left[t\right],\dot{q}\left[t\right]\right)-\left({x}_{G , i}, {y}_{G , j}\right)\right‖ ,$ wobei **(*i_{best},* (*j_{best}*)** den Index des zum Zeitpunkt ***t*** besten Kalibrierpunktes bezeichnet.

Alternativ oder ergänzend kann eine Inverse-Abstands (fachsprachlich: Inverse Distance) Interpolation aller Kalibrierdaten ausgeführt werden.

Zwar ist der Nächste-Nachbar-Ansatz einfach zu implementieren, doch entsteht in der Praxis ein Nachteil möglicherweise ein Nachteil, da die Kalibrierbilder im schlimmsten Fall ständig ausgetauscht werden. Dadurch kann es zu einem Flackern kommen, oder, falls ein langsamer Übergang von einem Kalibrationspunkt zum nächsten erfolgt, zunächst eine Verschlechterung der Bildqualität, bis der Kalibrationspunkt springt (z.B., da argmin in Gleichung (3) unstetig ist). Das Flackern und/oder Springen kann die medizinische Bildgebungsqualität negativ beeinflussen.

Alternativ oder ergänzend kann daher ein gewichteter Interpolationsansatz gewählt werden, der die einzelnen, zur Kompensation verwendeten Kalibrationsbilder stetig ineinander überführt. Hierzu kann die Distanz der geschätzten Auswanderung (wie beispielhaft in Fig. 8 an Bezugszeichen 806 gezeigt) zu jedem der Punkte bestimmt ${d}_{\left(i, j\right)} = \hat{f} \left(q\left[t\right],\dot{q}\left[t\right]\right) - \left({x}_{G , i}, {y}_{G , j}\right)$ werden und invers für einen gewichteten Mittelwert verwendet werden. Das Gewicht kann für alle ***d*_{(*i,j*)} ≠ 0** durch $w \left(i, j\right) = \frac{1}{{d}_{\left(i, j\right)}^{p}}$ (5) bestimmt (sog. Shepard's Weighting) sein, wobei ***p* ≥ 1** ein Parameter ist. Wenn mit ***B*(*i, j*)** die Menge der Kalibrationsdaten an jedem Kalibrationspunkt **(*i*, *j*)** bezeichnet wird, können die interpolierten Kalibrationsdaten gegeben sein durch $B = \left\{\begin{matrix}\frac{{\sum }_{\left(i, j\right)} {w}_{\left(i, j\right)} {B}_{\left(i, j\right)}}{{\sum }_{\left(i, j\right)} {w}_{\left(i, j\right)}} falls ∀ \left(i, j\right) : 0 < d \left(i, j\right) \leq ϵ , \\ {B}_{\left(i, j\right)} falls d \left(i, j\right) = 0 für ein \left(i, j\right) ,\end{matrix}\right.$ (6) wobei ***ε* > 0** ein Parameter ist, um die betrachtete Distanz auf einige nahe Punkte zu beschränken.

Der gewichtete Interpolationsansatz kann etwas rechenaufwendiger sein als der Nächste-Nachbar-Ansatz, dafür jedoch einen glatten (und idealerweise nicht merkbaren) Übergang zwischen den verschiedenen Kalibrierpunkten ermöglichen.

Eine Prozess-Sicht und/ein zeitlicher Ablauf um Training und Inferenz (auch: Ausführung) des NN kann wie folgt sein. Für jedes zu entwickelnde Röntgengerät und/oder System, bei dem die erfindungsgemäße Technik zum Einsatz kommt, wird in einem ersten Schritt in der Entwicklung des Röntgengeräts und/oder des Systems durch Simulation die Menge ***Uₛᵢₘ*** bestimmt und/oder durch Experiment an einem Versuchsträger die Menge ***Uₑₓₚ*** bestimmt.

In einem zweiten Schrittwird mit den Daten ***U = Uₛᵢₘ* U *Uₑₓₚ*** das NN, insbesondere gemäß einer Ausführungsbeispiel-Architekturen, grundlegend trainiert. Hierbei wird insbesondere ein overfitting der Daten vermieden.

In einem optionalen dritten Schritt werden für jedes einzelne Röntgengerät, das in der Fabrik gefertigt wird, weitere Experimentaldaten ***U_{exp,Instanz}*** gesammelt. Mit den Daten ***U_{exp,Instanz}*** kann ein fine-tuning des anhand des zweiten Schritts trainierten NNs. Das aus dem fine-tuning erhaltene Netz kann speziell für das einzelne Röntgengerät, das in der Fertigung entstanden ist, angepasst sein.

In einem vierten Schritt kann das trainierte NN mit einer Recheneinheit des Röntgengeräts zusammen an einen Kunden (z.B. eine Arztpraxis und/oder Krankenhaus) ausgeliefert werden und während der Lebensdauer zur Berechnung der Kompensationsdaten gemäß Gleichung (6) eingesetzt werden.

Je nach Ausführungsbeispiel kann Gleichung (3) anhand der Sollgrößen ***q_{d}*** (insbesondere vorneweg) berechnet werden, z.B. für eine komplette Bewegung, bevor diese ausgeführt wird. In diesem Fall wird der (beispielsweise) gesamte prognostizierte Zeitverlauf der Gewichte gemäß Gleichung (5) in einer Speichereinheit zwischengespeichert und während der Ausführung der Bewegung zur Berechnung der Gleichung (6) nur noch im passenden Zeitschritt ausgelesen. Alternativ oder ergänzend kann zur Laufzeit (fachsprachlich: real-time) Gleichung (6) während jeder Bewegung anhand der Ist-Daten ***q*[*t*]** in jedem Zeitschritt ***t*** berechnet werden.

Die Inferenz (und/oder ein Deployment) des trainierten NN kann als Ausführung auf einem Prozessor (z.B. einer CPU) des Steuerungssystems des Röntgengeräts (als ein mechatronisches medizinisches Gerät) erfolgen. Diese Ausführung kann etwas langsamer sein und daher insbesondere für die Variante der Vorabberechnung mittels Sollgrößen geeignet sein.

Alternativ oder ergänzend kann die Inferenz (und/oder ein Deployment) des trainierten NN ein Ausführung auf einer NPU oder GPU, speziell für Anwendung von NNs, umfassen, die beispielsweise auf einer Platine (und/oder einem Board) in der Steuereinheit eingebettet sein kann. Diese Ausführung kann gut geeignet sein für die Berechnung zur Laufzeit anhand von Ist-Daten.

Weiterhin alternativ oder ergänzend kann die Inferenz (und/oder ein Deployment) des trainierten NN eine Ausführung auf einem GPU-Cluster und/oder einem sonstigen Rechencluster umfassen, der aus dem lokalen Röntgengerät (und/oder einer lokalen Rechenvorrichtung) herausgeführt und/oder z.B. für eine AI-basierte Auswertung und Annotierung der medizinischen Bildgebungsdaten geeignet ist. Eine solcher Cluster kann vor Ort beim Kunden (z.B. in einer Arztpraxis und/oder einem Krankenhaus) stehen (on-premise) und/oder Cloud-basiert zur Verfügung gestellt werden. Diese Ausführung ist insbesondere für die Verwendung von Sollgrößen zur Vorabberechnung geeignet.

Soweit nicht bereits explizit beschrieben, können einzelne Ausführungsformen oder deren einzelne Aspekte und Merkmale, die in Bezug auf die Zeichnungen beschrieben sind, miteinander kombiniert oder ausgetauscht werden, ohne den Umfang der beschriebenen Erfindung einzuschränken oder zu erweitern, wenn eine solche Kombination oder ein solcher Austausch sinnvoll und im Sinne der vorliegenden Erfindung ist. Vorteile, die in Bezug auf eine bestimmte Ausführungsform der vorliegenden Erfindung oder in Bezug auf eine bestimmte Figur beschrieben sind, sind, wo immer dies zutrifft, auch Vorteile anderer Ausführungsformen der vorliegenden Erfindung.

Unabhängig vom grammatikalischen Geschlecht eines bestimmten Begriffes sind Personen mit männlicher, weiblicher oder anderer Geschlechteridentität mit umfasst.

### Zitierter Stand der Technik

[1] DE 10 2016 210 529 A1: Zeidler, Weber, Krämer: Mechanik zur Ausrichtung von Streustrahlengittern

## Patentansprüche

1. Computer-implementiertes Verfahren (100) zum Kompensieren, insbesondere in Echtzeit, eines Schattens eines Röntgenstreustrahlenabsorptionsgitters in einem Röntgenbild, das mit einem schwenkbar montierten Röntgengerät aufgenommen wird, umfassend die Verfahrensschritte:
- Empfangen (S102) von Bewegungszustandsdaten, die auf einen Bewegungszustand eines Röntgengeräts hinweisen, wobei das Röntgengerät auf einem schwenkbaren Arm montiert ist, und wobei der Bewegungszustand einem Zeitpunkt eines Aufnehmens eines Röntgenbilds mittels des Röntgengeräts zugeordnet ist;
- Bestimmen (S104) einer Position eines Röntgenstreustrahlenabsorptionsgitters, das am Detektor des Röntgengeräts angeordnet ist, wobei die Position des Röntgenstreustrahlenabsorptionsgitters eine Position relativ zur Röntgenquelle des Röntgengeräts umfasst, und wobei das Bestimmen (S104) in Abhängigkeit der empfangenen (S102) Bewegungszustandsdaten mittels eines, insbesondere datengetriebenen, Funktionsapproximators ausgeführt wird; und
- Kompensieren (S106), insbesondere in Echtzeit, eines Schattens des Röntgenstreustrahlenabsorptionsgitters auf dem aufgenommenen Röntgenbild, wobei das Kompensieren (S106) ein Berücksichtigen der bestimmten (S104) Position des Röntgenstreustrahlenabsorptionsgitters in dem aufgenommenen Röntgenbild umfasst, **dadurch gekennzeichnet, dass** die bestimmte Position des Röntgenstreustrahlenabsorptionsgitters unter Berücksichtigung einer relativen Verschiebung zwischen Röntgenquelle und Detektor bestimmt wird, die durch dynamische Kräfte, umfassend Trägheitskraft, Zentrifugalkraft und/oder Corioliskraft, hervorgerufen ist, wobei der Funktionsapproximator ein Modell f umfasst, welches eine direkte Abbildung des kinetischen Gerätezustands auf eine für die Verschiebung relevante Ebene des Röntgengitters bereitstellt.

2. Verfahren (100) gemäß Anspruch 1, wobei der schwenkbare Arm einen C-Bogen umfasst.

3. Verfahren (100) gemäß einem der vorhergehenden Ansprüche, wobei die Position des am Detektor angeordneten Röntgenstreustrahlenabsorptionsgitters eine Position in einer Detektorebene, und/oder in einer Ebene senkrecht zu einer Fokusrichtung der Röntgenquelle des Röntgengeräts, umfasst.

4. Verfahren (100) gemäß einem der vorhergehenden Ansprüche, wobei der Bewegungszustand eine Position, insbesondere eine Orientierung im Raum, und optional eine Geschwindigkeit einer Positionsverstellung, pro Freiheitsgrad des schwenkbaren Arms, und/oder des Röntgengeräts, umfasst.

5. Verfahren (100) gemäß einem der vorhergehenden Ansprüche, wobei der Funktionsapproximator eine Abbildung von einem mindestens M-dimensionalen, insbesondere 2M-dimensionalen, Bewegungszustand des Röntgengeräts auf eine zwei-dimensionale, 2D, Position des am Detektor angeordneten Röntgenstreustrahlenabsorptionsgitters umfasst, wobei M eine Anzahl unabhängiger, insbesondere schwenkbarer, Freiheitsgrade des schwenkbaren Arms des Röntgengeräts umfasst.

6. Verfahren (100) gemäß einem der vorhergehenden Ansprüche, wobei der Funktionsapproximator ein trainiertes Neuronales Netzwerk, NN, umfasst, wobei:
- Eine Anzahl Neuronen einer Eingangsschicht des NN ein ganzzahliges Vielfaches einer Anzahl M unabhängig positionsverstellbarer Freiheitsgrade des Röntgengeräts umfasst, wobei das ganzzahlige Vielfache vorzugsweise gleich eins oder zwei ist; und wobei
- Eine Ausgangsschicht des NN zwei Neuronen umfasst, deren Ausgabe jeweils einer Richtung einer Detektorebene zugeordnet ist.

7. Verfahren (100) gemäß dem direkt vorhergehenden Anspruch, wobei das NN mittels überwachtem Training trainiert ist, wobei ein Trainingsdatensatz umfasst:
- Eingangsdaten, die Bewegungszustandsdaten des Röntgengeräts umfassen; und
- Ausgangsdaten, die eine vom Bewegungszustand des Röntgengeräts abhängige Position des Röntgenstreustrahlenabsorptionsgitters umfassen.

8. Verfahren (100) gemäß dem direkt vorhergehenden Anspruch, wobei die Trainingsdatensätze ausgewählt sind aus der Gruppe:
- Realer Trainingsdatensätze, wobei die realen Trainingsdatensätze Messwerte hinweisend auf den Bewegungszustand und auf die Position des Röntgenstreustrahlenabsorptionsgitters umfassen; und/oder
- Synthetische Trainingsdatensätze, wobei die synthetischen Trainingsdatensätze synthetisch generierte Daten hinweisend auf den Bewegungszustand sowie eine mittels eines Simulationsmodells bestimmte Position des Röntgenstreustrahlenabsorptionsgitters umfassen.

9. Verfahren (100) gemäß einem der Ansprüche 6 bis 8, wobei das NN eine Feedforward NN, FNN, Architektur umfasst, optional wobei das FNN Schichten als Multi-Layer-Perzeptron, MLP, umfasst.

10. Verfahren (100) gemäß einem der Ansprüche 6 bis 9, wobei das NN eine Rekurrente NN, RNN, Architektur umfasst, optional wobei das RNN eine Long Short-Term Memory, LSTM, Architektur umfasst.

11. Verfahren (100) gemäß einem der vorhergehenden Ansprüche, ferner umfassend den Schritt des:
- Ausgebens (S108) des aufgenommenen Röntgenbilds umfassend den, insbesondere in Echtzeit, kompensierten (S106) Schatten des Röntgenstreustrahlenabsorptionsgitters.

12. Verfahren (100) gemäß einem der vorhergehenden Ansprüche, wobei im Schritt des Bestimmens (S104), mittels des, insbesondere datengetriebenen, Funktionsapproximators, ein Kalibrierpunkt, und optional ein Kalibrierbild, mittels eines Optimierungsverfahrens bestimmt wird;
wobei weiterhin optional im Schritt des Kompensierens (S106), insbesondere in Echtzeit, des Schattens des Röntgenstreustrahlenabsorptionsgitters das Kalibrierbild mit dem aufgenommenen Röntgenbild verrechnet, insbesondere von dem aufgenommenen Röntgenbild subtrahiert, wird.

13. Rechenvorrichtung (200) zum Kompensieren, insbesondere in Echtzeit, eines Schattens eines Röntgenstreustrahlenabsorptionsgitters in einem Röntgenbild, das mit einem schwenkbar montierten Röntgengerät aufgenommen wird, umfassend:
- Eine Empfangsschnittstelle (202), die zum Empfangen von Bewegungszustandsdaten, die auf einen Bewegungszustand eines Röntgengeräts hinweisen, ausgebildet ist, wobei das Röntgengerät auf einem schwenkbaren Arm montiert ist, und wobei der Bewegungszustand einem Zeitpunkt eines Aufnehmens eines Röntgenbilds mittels des Röntgengeräts zugeordnet ist;
- Einen, insbesondere datengetriebenen, Funktionsapproximator (204), der zum Bestimmen einer Position eines Röntgenstreustrahlenabsorptionsgitters, das am Detektor des Röntgengeräts angeordnet ist, ausgebildet ist, wobei die Position des Röntgenstreustrahlenabsorptionsgitters eine Position relativ zur Röntgenquelle des Röntgengeräts umfasst, und wobei das Bestimmen in Abhängigkeit der empfangenen Bewegungszustandsdaten ausgeführt wird wobei die bestimmte Position des Röntgenstreustrahlenabsorptionsgitters unter Berücksichtigung einer relativen Verschiebung zwischen Röntgenquelle und Detektor bestimmt wird, die durch dynamische Kräfte, umfassend Trägheitskraft, Zentrifugalkraft und/oder Corioliskraft, hervorgerufen ist, wobei der Funktionsapproximator ein Modell f umfasst, welches eine direkte Abbildung des kinetischen Gerätezustands auf eine für die Verschiebung relevante Ebene des Röntgengitters bereitstellt. und
- Eine Kompensiereinheit (206), die zum Kompensieren, insbesondere in Echtzeit, eines Schattens des Röntgenstreustrahlenabsorptionsgitters auf dem aufgenommenen Röntgenbild, ausgebildet ist, wobei das Kompensieren ein Berücksichtigen der bestimmten Position des Röntgenstreustrahlenabsorptionsgitters in dem aufgenommenen Röntgenbild umfasst.

14. Rechenvorrichtung (200) gemäß dem direkt vorhergehenden Anspruch, wobei die Rechenvorrichtung (200) dazu ausgebildet ist, einen oder mehrere der Schritte der Ansprüche 2 bis 12 auszuführen.

15. System zum Kompensieren, insbesondere in Echtzeit, eines Schattens eines Röntgenstreustrahlenabsorptionsgitters in einem Röntgenbild, das mit einem schwenkbar montierten Röntgengerät aufgenommen wird, umfassend:
- ein auf einem schenkbaren Arm montiertes Röntgengerät mit einem Röntgenstreustrahlenabsorptionsgitter; und
- eine Rechenvorrichtung (200) gemäß Anspruch 13 oder 14.

16. Computerprogrammprodukt mit Programmelementen, die eine Rechenvorrichtung (200) veranlassen, die Schritte des Verfahrens zum Kompensieren, insbesondere in Echtzeit, eines Schattens eines Röntgenstreustrahlenabsorptionsgitters in einem Röntgenbild, das mit einem schwenkbar montierten Röntgengerät aufgenommen wird, nach einem der vorhergehenden Verfahrensansprüche auszuführen, wenn die Programmelemente in einen Speicher der Rechenvorrichtung (200) geladen werden.

17. Computerlesbares Medium, auf dem Programmelemente gespeichert sind, die von einer Rechenvorrichtung (200) gelesen und ausgeführt werden können, um Schritte des Verfahrens zum Kompensieren, insbesondere in Echtzeit, eines Schattens eines Röntgenstreustrahlenabsorptionsgitters in einem Röntgenbild, das mit einem schwenkbar montierten Röntgengerät aufgenommen wird, gemäß einem der vorangehenden Verfahrensansprüche durchzuführen, wenn die Programmelemente von der Rechenvorrichtung (200) ausgeführt werden.

## Claims

1. Computer-implemented method (100) for compensating, in particular in real-time, for a shadow of an x-ray scattered beam absorption grid in an x-ray image, which is recorded with a pivotably mounted x-ray device,
comprising the method steps:
- receiving (S102) state of movement data, which indicates a state of movement of an x-ray device, wherein the x-ray device is mounted on a pivotable arm, and wherein the state of movement is assigned to a point in time of recording an x-ray image by means of the x-ray device;
- determining (S104) a position of an x-ray scattered beam absorption grid, which is arranged on the detector of the x-ray device, wherein the position of the x-ray scattered beam absorption grid comprises a position relative to the x-ray source of the x-ray device, and wherein the determination (S104) is executed as a function of the received (S102) state of movement data by means of a functional approximator which is in particular data driven; and
- compensating (S106), in particular in real-time, for a shadow of the x-ray scattered beam absorption grid on the recorded x-ray image, wherein the compensation (S106) comprises taking into account the determined (S104) position of the x-ray scattered beam absorption grid in the recorded image, **characterised in that** the determined position of the x-ray scattered beam absorption grid is determined by taking into account a relative displacement between x-ray source and detector, which is provoked by dynamic forces comprising inertia force, centrifugal force and/or Coriolis force, wherein the functional approximator comprises a model f which provides a direct mapping of the kinetic device state on a plane of the x-ray grid which is relevant to the displacement.

2. Method (100) according to claim 1, wherein the pivotable arm comprises a C-arm.

3. Method (100) according to one of the preceding claims, wherein the position of the x-ray scattered beam absorption grid arranged on the detector comprises a position in a detector plane, and/or in a plane at a right angle to a focus direction of the x-ray source of the x-ray device.

4. Method (100) according to one of the preceding claims, wherein the state of movement comprises a position, in particular an orientation in the room, and optionally a speed of a position adjustment, per degree of freedom of the pivotable arm, and/or the x-ray device.

5. Method (100) according to one of the preceding claims, wherein the functional approximator comprises a mapping of one at least M-dimensional, in particular 2M-dimensional, state of movement of the x-ray device on a two-dimensional, 2D, position of the x-ray scattered beam absorption grid arranged on the detector, wherein M comprises a number of independent, in particular pivotable, degrees of freedom of the pivotable arm of the x-ray device.

6. Method (100) according to one of the preceding claims, wherein the functional approximator comprises a trained neural network, NN, wherein:
- a number of neurons of an input layer of the NN comprises a whole-number multiple of a number M of independent position-adjustable degrees of freedom of the x-ray device, wherein the whole-number multiple is preferably equal to one or two; and wherein
- an output layer of the NN comprises two neurons, the output of which is assigned in each case to a direction of a detector plane.

7. Method (100) according to the directly preceding claim, wherein the NN is trained by means of supervised training, wherein a training dataset comprises:
- input data, which comprise state of movement data of the x-ray device; and
- output data, which comprises a position of the x-ray scattered beam absorption grid which is dependent on the state of movement of the x-ray device.

8. Method (100) according to the directly preceding claim, wherein the training data sets are selected from the group:
- real training data sets, wherein the real training data sets comprise measured values indicating the state of movement and the position of the x-ray scattered beam absorption grid; and/or
- synthetic training data sets, wherein the synthetic training datasets comprise synthetically generated data indicating the state of movement and a position of the x-ray scattered beam absorption grid determined by means of a simulation model.

9. Method (100) according to one of claims 6 to 8, wherein the NN comprises a feedforward NN, FNN, architecture, optionally wherein the FNN comprises layers as a multilayer perceptron, MLP.

10. Method (100) according to one of claims 6 to 9, wherein the NN comprises a recurrent NN, RNN, architecture, optionally wherein the RNN comprises a long short-term memory, LSTM, architecture.

11. Method (100) according to one of the preceding claims, further comprising the step of:
- outputting (S108) the recorded x-ray image comprising the shadow of the x-ray scattered beam absorption grid which is compensated (S106) in particular in real-time.

12. Method (100) according to one of the preceding claims, wherein in the step of determining (S104), by means of the, in particular data-driven, functional approximator, a calibration point, and optionally a calibration image, is determined by means of an optimization method;
wherein furthermore optionally in the step of compensating (S106), in particular in real-time, for the shadow of the x-ray scattered beam absorption grid, the calibration image is calculated with the recorded x-ray image, in particular subtracted from the recorded x-ray image.

13. Computing apparatus (200) for compensating, in particular in real-time, for a shadow of an x-ray scattered beam absorption grid in an x-ray image, which is recorded with a pivotably mounted x-ray device, comprising:
- a receiving interface (202) which is embodied to receive state of movement data, which indicates a state of movement of an x-ray device, wherein the x-ray device is mounted on a pivotable arm and wherein the state of movement is assigned to a point in time of recording an x-ray image by means of the x-ray device;
- a, in particular data-driven, functional approximator (204), which is embodied to determine a position of an x-ray scattered beam absorption grid, which is arranged on the detector of the x-ray device, wherein the position of the x-ray scattered beam absorption grid comprises a position relative to the x-ray source of the x-ray device, and wherein the determination is executed as a function of the received state of movement data, wherein the determined position of the x-ray scattered beam absorption grid is determined by taking into account a relative displacement between x-ray source and detector, which is provoked by dynamic forces, comprising inertia force, centrifugal force and/or Coriolis force, wherein the functional approximator comprises a model f, which provides a direct mapping of the kinetic device state on a plane of the x-ray grid which is relevant to the displacement, and
- a compensation unit (206), which is embodied to compensate, in particular in real-time, for a shadow of the x-ray scattered beam absorption grid on the recorded x-ray image, wherein the compensation comprises taking into account the determined position of the x-ray scattered beam absorption grid in the recorded x-ray image.

14. Computing apparatus (200) according to the directly preceding claim, wherein the computing apparatus (200) is embodied to execute one or more of the steps of claims 2 to 12.

15. System for compensating, in particular in real-time, for a shadow of an x-ray scattered beam absorption grid in an x-ray image, which is recorded with a pivotably mounted x-ray device, comprising:
- an x-ray device mounted on a pivotable arm with an x-ray scattered beam radiation absorption grid; and
- a computing apparatus (200) according to claim 13 or 14.

16. Computer program product with program elements, which provoke a computing apparatus (200) to execute the steps of the method for compensating, in particular in real-time, for a shadow of an x-ray scattered beam absorption grid in an x-ray image, which is recorded with a pivotably mounted x-ray device, according to one of the preceding method claims, when the program elements are loaded into a memory of the computing apparatus (200).

17. Computer-readable medium, on which program elements are stored, which can be read and executed by a computing apparatus (200) in order to carry out steps of the method for compensating, in particular in real-time, for a shadow of an x-ray scattered beam absorption grid in an x-ray image, which is recorded with a pivotably mounted x-ray device, according to one of the preceding method claims, when the program elements are executed by the computing apparatus (200).

## Revendications

1. Procédé (100) mis en œuvre par ordinateur de compensation, en particulier en temps réel, de l'ombre d'un réseau d'absorption de rayons X de dispersion dans une image de rayons X, qui est prise par un appareil de rayons X monté pivotant, comprenant les stades de procédé :
- réception (S102) de données d'état de mouvement, qui indiquent un état de mouvement d'un appareil de rayons X, dans lequel l'appareil de rayons X est monté sur un bras pivotant, et dans lequel l'état de mouvement est associé à un instant d'une prise d'une image de rayons X au moyen de l'appareil de rayons X ;
- détermination (S104) d'une position d'une grille d'absorption de rayons X de dispersion, qui est montée sur le détecteur de l'appareil de rayons X, dans lequel la position de la grille d'absorption de rayons X de dispersion comprend une position par rapport à la source de rayons X de l'appareil de rayons X, et dans lequel on effectue la détermination (S104) en fonction des données d'état de mouvement reçues (S102) au moyen d'un approximateur de fonction, en particulier fondé sur des données ; et
- compensation (S106), en particulier en temps réel, de l'ombre de la grille d'absorption de rayons X de dispersion sur l'image de rayons X prise, dans lequel la compensation (S106) comprend une prise en compte de la position déterminée (S104) de la grille d'absorption de rayons X de dispersion dans l'image de rayons X prise, **caractérisé en ce que** l'on détermine la position déterminée de la grille d'absorption de rayons X de dispersion en tenant compte d'un . déplacement relatif entre source de rayonnement et détecteur, qui est provoqué par des forces dynamiques, comprenant force d'inertie, force centrifuge et/ou force de Coriolis, dans lequel l'approximateur de fonction comprend un modèle f, qui donne une représentation directe de l'état cinétique de l'appareil sur un plan de la grille de rayons X pertinent pour le déplacement.

2. Procédé (100) suivant la revendication 1, dans lequel le bras pivotant comprend un arceau en C.

3. Procédé (100) suivant l'une des revendications précédentes, dans lequel la position de la grille d'absorption de rayons X de dispersion montée sur le détecteur comprend une position dans un plan du détecteur, et/ou dans un plan perpendiculaire à une direction focale de la source de rayons X de l'appareil de rayons X.

4. Procédé (100) suivant l'une des revendications précédentes, dans lequel l'état de mouvement comprend une position, en particulier une orientation dans l'espace, et éventuellement une vitesse d'un déplacement de position, par degré de liberté du bras pivotant et/ou de l'appareil de rayons X.

5. Procédé (100) suivant l'une des revendications précédentes, dans lequel l'approximateur de fonction comprend une représentation d'un état de mouvement au moins à M dimensions, en particulier à 2M dimensions, de l'appareil de rayons X sur une position en deux dimensions, 2D, de la grille d'absorption de rayons X de dispersion montée sur le détecteur, M comprenant un nombre de degrés de liberté indépendants, en particulier orientables, du bras pivotant de l'appareil de rayons X. .

6. Procédé (100) suivant l'une des revendications précédentes, dans lequel l'approximateur de fonction comprend un réseau neuronal entraîné, NN, dans lequel :
- un nombre de neurones d'une couche d'entrée du NN comprend un multiple en nombre entier d'un nombre M de degrés de liberté, réglables en position indépendamment, de l'appareil de rayons X, dans lequel le multiple en nombre entier est de préférence égal à un ou à deux ; et dans lequel
- une couche de sortie du NN comprend deux neurones, dont la sortie est associée respectivement à une direction d'un plan du détecteur.

7. Procédé (100) suivant la revendication directement précédente, dans lequel le NN est entraîné au moyen d'un entraînement contrôlé, dans lequel un ensemble de données d'entraînement comprend :
- des données d'entrée, qui comprennent les données d'état de mouvement de l'appareil de rayons X ; et
- des données de sortie, qui comprennent une position, qui dépend de l'état de mouvement de l'appareil de rayons X, de la grille d'absorption de rayons X de dispersion.

8. Procédé (100) suivant la revendication directement précédente, dans lequel les ensembles de données d'entraînement sont sélectionnés dans le groupe :
- ensembles de données d'entraînement réels, dans lequel les ensembles de données d'entraînement réels comprennent des valeurs de mesure indiquant l'état de déplacement et la position de la grille d'absorption de rayons X de dispersion ; et/ou
- ensembles de données d'entraînement synthétiques, dans lequel les ensembles de données d'entraînement synthétiques comprennent des données créées synthétiquement indiquant . l'état de déplacement ainsi qu'une position, déterminée au moyen d'un modèle de simulation, de la grille d'absorption de rayons X de dispersion.

9. Procédé (100) suivant l'une des revendications 6 à 8, dans lequel le NN comprend une architecture feedforward NN, FNN, dans lequel éventuellement le FNN comprend des couches sous la forme de perceptron multicouche, MLP.

10. Procédé (100) suivant l'une des revendications 6 à 9, dans lequel le NN comprend une architecture NN récurrente, RNN, dans lequel éventuellement le RNN comprend une architecture long-short-term memory, LSTM.

11. Procédé (100) suivant l'une des revendications précédentes, comprenant en outre le stade de :
- la sortie (S108) de l'image de rayons X prise comprenant, en particulier en temps réel, l'ombre compensée (S106) de la grille d'absorption de rayons X de dispersion.

12. Procédé (100) suivant l'une des revendications précédentes, dans lequel dans le stade de la détermination (S104), au moyen de l'approximateur de fonction, en particulier fondé sur des données, on détermine un point d'étalonnage, et éventuellement une image d'étalonnage, au moyen d'un procédé d'optimisation ;
dans lequel en outre éventuellement dans le stade de la compensation (S106), en particulier en temps réel, de l'ombre de la grille d'absorption de rayons X de dispersion, on calcule l'image d'étalonnage avec l'image de rayons X prise, en particulier par soustraction de l'image de rayons X prise.

13. Dispositif (200) informatique de compensation, en particulier en temps réel, de l'ombre d'une grille d'absorption . de rayons X de dispersion dans une image de rayons X, qui est prise par un appareil de rayons X monté pivotant, comprenant :
- une interface (202) de réception, qui est constituée pour la réception de données d'état de mouvement, qui indiquent un état de mouvement d'un appareil de rayons X, dans lequel l'appareil de rayons X est monté sur un bras pivotant, et dans lequel l'état de mouvement est associé à un instant d'une prise d'une image de rayons X au moyen de l'appareil de rayons X ;
- un approximateur (204) de fonction, en particulier fondé sur des données, qui est constitué pour la détermination d'une position d'une grille d'absorption de rayons X de dispersion, qui est montée sur le détecteur de l'appareil X, dans lequel la position de la grille d'absorption de rayons X de dispersion comprend une position par rapport à la source de rayons X de l'appareil de rayons X, et dans lequel on effectue la détermination en fonction de données d'état de mouvement reçues, dans lequel on détermine la position déterminée de la grille d'absorption de rayons X de dispersion en tenant compte d'un déplacement relatif entre source de rayonnement et détecteur, qui est provoqué par des forces dynamiques, comprenant force d'inertie, force centrifuge et/ou force de Coriolis, dans lequel l'approximateur de fonction comprend un modèle f, qui donne une représentation directe de l'état cinétique de l'appareil sur un plan de la grille de rayons X pertinent pour le déplacement, et
- une unité (206) de compensation, qui est constituée pour compenser, en particulier en temps réel, l'ombre de la grille d'absorption de rayons X de dispersion sur l'image de rayons X prise, dans lequel la compensation comprend une prise en compte de la position déterminée de la grille d'absorption de rayons X de dispersion dans l'image de rayons X prise.
.

14. Dispositif (200) informatique suivant la revendication directement précédente,
dans lequel
le dispositif (200) informatique est constitué pour effectuer un ou plusieurs des procédés des revendications 2 à 12.

15. Système de compensation, en particulier en temps réel, de l'ombre d'une grille d'absorption de rayons X de dispersion dans une image de rayons X, qui est prise par un appareil de rayons X monté pivotant, comprenant :
- un appareil de rayons X monté sur un bras pivotant et ayant une grille d'absorption de rayons X de dispersion ; et
- un dispositif (200) informatique suivant la revendication 13 ou 14.

16. Produit de programme d'ordinateur, comprenant des éléments de programme, qui font qu'un dispositif (200) informatique effectue le stade du procédé de compensation, en particulier en temps réel, de l'ombre d'une grille d'absorption de rayons X de dispersion dans une image de rayons X, qui est prise par un appareil de rayons X monté pivotant, suivant l'une des revendications précédentes de procédé, lorsque les éléments de programme sont chargés dans une mémoire du dispositif (200) informatique.

17. Support déchiffrable par ordinateur, sur lequel sont mis en mémoire des éléments de programme, qui peuvent être lus et exécutés par un dispositif (200) informatique pour effectuer tous les stades du procédé de compensation, en particulier en temps réel, de l'ombre d'une grille d'absorption de rayons X de dispersion dans une image de rayons X, qui est prise par un appareil de rayons X monté pivotant, suivant l'une des revendications précédentes de . procédé, lorsque les éléments de programme sont exécutés par le dispositif (200) informatique.
